# EUROPEAN PATENT SPECIFICATION

(11) **EP 3 845 636 B1**
(45) Date of publication and mention of the grant of the patent: **31.07.2024**
(21) Application number: 19854133.6
(22) Date of filing: 30.08.2019
(51) Int. Cl.: C12N 5/0775, C12P 1/00, C12N 5/071, C12P 21/00, C12N 5/00

(54) **MEDIUM COMPOSITION FOR SUSPENSION CULTURE OF ADHESIVE CELLS**
MEDIENZUSAMMENSETZUNG FÜR DIE SUSPENSIONSKULTUR VON ADHÄSIVEN ZELLEN
COMPOSITION DE MILIEU POUR LA CULTURE EN SUSPENSION DE CELLULES ADHÉSIVES

(30) Priority: 31.08.2018 JP 2018164042; 19.07.2019 JP 2019134058
(43) Date of publication of application: 07.07.2021
(73) Proprietor: Nissan Chemical Corporation, Tokyo 103-6119 (JP)
(72) Inventor: KANAKI, Tatsuro, Shiraoka-shi, Saitama 349-0294 (JP); KIDA, Katsuhiko, Shiraoka-shi, Saitama 349-0294 (JP); MINAMI, Masataka, Funabashi-shi, Chiba 274-0052 (JP); HATANAKA, Daisuke, Funabashi-shi, Chiba 274-0052 (JP)
(74) Representative: J A Kemp LLP
(86) International application number: PCT/JP2019/034099
(87) International publication number: WO 2020/045620

(56) References cited:
- EP-A1- 3 098 300
- WO-A1-2015/111686
- WO-A1-2017/154952
- WO-A1-2017/175751
- WO-A1-2017/175751
- KIDA KATSUHIKO ET AL: "A Novel 3D Culture System Using a Chitin-Based Polysaccharide Material Produces High-Quality Allogeneic Human UCMSCs with Dispersed Sphere Morphology", CELLS, vol. 11, no. 6, 15 March 2022 (2022-03-15), pages 995, XP055971274, DOI: 10.3390/cells11060995
- KIM EUN SEO ET AL: "Cellhesion VP enhances the immunomodulating potential of human mesenchymal stem cell-derived extracellular vesicles", BIOMATERIALS, vol. 271, 1 April 2021 (2021-04-01), AMSTERDAM, NL, pages 120742, XP055971276, ISSN: 0142-9612, DOI: 10.1016/j.biomaterials.2021.120742
- MORIMOTO, MINORU, SHIGEMASA, YOSHIHIRO, ": "Characterization and Bioactivities of Chitin and Chitosan Regulated Their Degree of Deacetylation", KOBUNSHI RONBUNSHU, vol. 54, no. 10, 1997, pages 621 - 631, XP000755555
- HUSSAIN, ALI ET AL.: "Functional 3-D Cardiac Co-Culture Model Using Bioactive Chitosan Nanofiber Scaffolds", BIOTECHNOL. BIOENG., vol. 110, 2013, pages 637 - 647, XP055214701

## Description

### [Technical Field]

The present invention relates to a medium composition for suspension culture of adherent cells, and a method for suspension culture of adherent cells. More particularly, the present invention relates to a medium composition for suspension culture of adherent cells containing chitin nanofiber and chitosan nanofiber and, a method for suspension culture of adherent cells using the medium composition.

### [Background Art]

In the field of regenerative medicine, construction of organ regeneration means using pluripotent stem cells such as iPS cells and ES cells has been intensively studied. However, there is an ethical problem in the establishment of ES cells, and a tumorigenicity risk and the length of culture period have been recognized as problems of iPS cells. Thus, the possibility of means using somatic stem cells such as mesenchymal stem cells and neural stem cells that have a comparatively low tumorigenicity risk, precursor cells such as pre-adipocytes and progenitor cardiomyocytes having a comparatively short differentiation induction period, or chondrocytes and the like is also being explored in parallel.

Since therapeutic approaches using somatic stem cells and the like require a large amount of such cells with high quality, a culture method capable of efficiently preparing cells with high quality has been demanded. For example, it is known that mesenchymal stem cells can be relatively easily proliferated in a petri dish by monolayer culture (also referred to as two-dimensional (2D) culture or the like).

However, the number of cells that can be cultured in one petri dish is limited. When culturing a large amount of cells using this method, it is necessary to consider stacking many petri dishes, or the like. Accordingly, this culture method is not suitable from the viewpoint of large-scale production. According to recent reports, two-dimensional culture methods using petri dish may reduce the undifferentiation potency and proliferative capacity of mesenchymal stem cells. In addition, a decline in the functions of mesenchymal stem cells, such as chemotactic activity including homing action, anti-inflammatory action, and the like is also feared. Therefore, the two-dimensional culture method using a petri dish is also unsuitable from the viewpoint of producing high-quality cells.

On the other hand, as a method for culturing a large amount of adherent cells, a device for culturing and proliferating cells while being adhered to a microcarrier has been reported (patent document 1). However, currently available microcarriers form sediment in the culture medium under static conditions, and need to be stirred during culture. A problem has been pointed out that cell death occurs due to collision between carriers during the stirring. Furthermore, when cells are recovered from microcarriers for the purpose of media change, scale-up of culture or the like, it is necessary to detach a cell from another cell or a cell from a carrier. One of the problems here is that cells are damaged by proteases such as trypsin and the like used in the detaching treatment, and as a result, the survival rate of cells is reduced.

### [Document List]

### [Patent document]

patent document 1: JP-B-3275411
EP3098300 relates to a culture method of cells and/or tissues including culturing cells and/or tissues in a suspended state by using a medium composition having the effect of preventing sedimentation of cells and/or tissues.
WO2017/175751 relates to a method for producing proteins in which cells have protein-producing capability are suspension cultured, under physically agitated conditions, in a medium composition containing nanofibers, said cells being adhered to the nanofibers.

### [Summary of Invention]

### [Technical Problem]

From the aforementioned background, the problem of the present invention is to provide a new culture method that can solve plural problems in the conventional suspension culture of adherent cells using a microcarrier, such as cell death due to collision of microcarriers during stirring, cell damage due to protease treatment during passage culture, and the like.

The present inventors have conducted intensive studies in an attempt to solve the aforementioned problems and found that blending chitin nanofiber and chitosan nanofiber or specific polysaccharides in a specific ratio in a liquid medium results in the following: (1) adherent cells can be suspension cultured in the liquid medium and can be efficiently proliferated because stirring is not required, (2) a carrier and adherent cells adhering to the carrier can be easily redispersed by gently stirring the liquid medium, and suspension culture can be performed again continuously by allowing the liquid medium to stand still, (3) the stem cells (e.g., mesenchymal stem cells) proliferated in the liquid medium maintain very high levels of differentiation potency and chemotactic activity, (4) using the liquid medium, cells can be maintained and cultured for a long time under low serum conditions, and a cell secretion product can be efficiently produced, (5) the dispersibility of the sphere can be maintained for a long period of time by using the liquid medium, and the like. Based on such findings, they have conducted further studies and completed the present invention. That is, the present invention provides the following.

The invention provides a medium composition for suspension culture of a mesenchymal stem cell, comprising
(1) a chitin nanofiber; and
(2) a chitosan nanofiber;

wherein the ratio of the chitin nanofiber and the chitosan nanofiber is chitin nanofiber:chitosan nanofiber = 1:3 - 6; wherein the ratio of N-acetylglucosamine in the sugar units constituting chitin is not less than 90%; and
wherein the ratio of N-acetylglucosamine in the sugar units constituting chitosan is not more than 30%. In an embodiment, the mesenchymal stem cell is a cell that self-aggregates under suspension culture. In an embodiment, the suspension culture is for proliferating the mesenchymal stem cell, and maintaining pluripotency and chemotacticity of the mesenchymal stem cell.

The invention also provides a method for culturing a mesenchymal stem cell, comprising a step of suspension culturing the mesenchymal stem cell in a medium composition of the invention. In an embodiment, the method is one further comprising the following steps: (1) a step of adding the medium composition of the invention without performing a treatment for detaching the suspension cultured cells from the chitin nanofiber, and chitosan nanofiber, and (2) a step of subjecting the mixture obtained in step (1) to suspension culture.

The invention additionally provides a method for producing a cell secretion product, comprising a step of suspension culturing a mesenchymal stem cell in a medium composition of the invention. In an embodiment, a concentration of the serum in the medium composition is not more than 2%. In an embodiment, the cell secretion product is at least one selected from the group consisting of a low-molecular-weight compound, a protein, a nucleic acid, and a cell secretion vesicle.

### [Advantageous Effects of Invention]

As described herein, adherent cells can be suspension cultured in a stationary state by culturing them while being attached to nanofibers composed of water-insoluble polysaccharides, without an operation such as shaking, rotation and the like having a risk of causing injury and loss of functions of cells. The adherent cell cultured using the present invention is a mesenchymal stem cell, and a high-quality stem cell that maintains high levels of properties as a stem cell (e.g., differentiation potency, chemotactic/homing activity etc. in mesenchymal stem cells) can be prepared efficiently. According to the present invention, moreover, the culture can be easily scaled up by only gently stirring by pipetting and the like and adding a fresh medium, without performing an operation using a protease such as trypsin and the like to detach a carrier and the cells adhering thereto. As a result, a useful cell secretion product can also be produced efficiently.

### [Brief Description of Drawings]

Fig. 1 is a graph showing the number of viable cells over time when HEK293-IFNβ cells were suspension cultured for 21 days using the medium composition added with sample 1 (chitin nanofiber), sample 6 (mixture of chitin nanofiber and chitosan nanofiber), sample 14 (chitin/chitosan nanofiber prepared by simultaneous fibrillating), or sample 15 (nanofiber adjusted in the amount of N-acetylglucosamine (Nacetylglucosamine: 50%)). The four bar graphs for each sample show the number of viable cells on days 0, 7, 14, and 21 from the left, respectively.
Fig. 2 is a photograph showing the sphere state (day 8) of cells when HEK293-IFNβ cells were suspension cultured using a medium composition added with each nanofiber.
Fig. 3 is a photograph showing the sphere state (day 15) of cells when HEK293-IFNβ cells were suspension cultured using a medium composition added with each nanofiber.
Fig. 4 is a photograph showing the sphere state (day 21) of cells when HEK293-IFNβ cells were suspension cultured using a medium composition added with each nanofiber.

### [Description of Embodiments]

The present invention is described in detail in the following.

### 1. Medium composition for suspension culture of adherent cells

The present invention provides a medium composition for suspension culture of a mesenchymal stem cell containing (1) a chitin nanofiber; and (2) a chitosan nanofiber wherein the ratio of the chitin nanofiber and the chitosan nanofiber is chitin nanofiber:chitosan nanofiber=1:3 - 6;
wherein the ratio of N-acetylglucosamine in the sugar units constituting chitin is not less than 90%; and
wherein the ratio of N-acetylglucosamine in the sugar units constituting chitosan is not more than 30%,(hereinafter sometimes referred to as "the medium composition of the present invention"). The medium composition of the present invention containing chitin nanofiber and chitosan nanofiber permits suspension culture of the adherent cells without requiring an operation such as stirring, shaking or the like.

The adherent cell used in the present invention is a cell that requires a scaffold such as a container wall and the like for survival and proliferation.

The adherent cell to be used in the present invention is mesenchymal stem cell. Mesenchymal stem cell is a stem cell having differentiation potency into all or some of osteocyte, chondrocyte and adipocyte. Mesenchymal stem cell is present in a tissue such as bone marrow, peripheral blood, cord blood, adipose tissue and the like at a low frequency and can be isolated from these tissues by a known method.

The derivation of the adherent cell to be used in the present invention is not particularly limited, and the cell may be derived from any animal or plant. Examples of the animal include insect, fish, amphibian, reptiles, birds, pancrustacea, hexapoda, mammals and the like, with preference given to mammal. Examples of the mammal include, but are not limited to, rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like. The plant is not particularly limited as long as the collected cells can be applied to liquid culture. Examples thereof include, but are not limited to, plants (e.g., ginseng, periwinkle, henbane, coptis, belladonna etc.) producing crude drugs (e.g., saponin, alkaloids, berberine, scopolin, phytosterol etc.), plants (e.g., blueberry, safflower, madder, saffron etc.) producing dye or polysaccharide (e.g., anthocyanin, safflower dye, madder dye, saffron dye, flavones etc.) to be a starting material for cosmetic or food, or plants producing a pharmaceutical active pharmaceutical ingredient, and the like. In the present invention, mammalian adherent cells are preferably used.

In one embodiment, the adherent cell may be an adherent cell that self-aggregates in suspension culture. The adherent cell that self-aggregates in suspension culture includes cells that form spheres in suspension culture. Adherent cells that self-aggregate in suspension culture include mesenchymal stem cells.

The chitin nanofiber, and chitosan nanofiber to be used in the present invention are dispersed in a liquid medium and show an effect of suspending adherent cells attached to the nanofiber in the liquid medium.

The chitin nanofiber, and chitosan nanofiber to be used in the present invention are, upon mixing with a liquid medium, dispersed in the liquid while maintaining the primary fiber diameter, substantially retains the cells attached to the nanofiber without substantially increasing the viscosity of the liquid, and shows an effect of preventing sedimentation and attachment thereof to the culture container. Without substantially increasing the viscosity of the liquid means that the viscosity of the liquid does not exceed 8 mPa·s. In this case, the viscosity of the liquid (that is, the viscosity of the following medium composition of the present invention) is not more than 8 mPa·s, preferably not more than 4 mPa·s, more preferably not more than 2 mPa·s. The viscosity of the liquid containing chitin nanofiber, and chitosan nanofiber can be measured, for example, using a tuning fork vibration type viscometer (SV-1A, A&D Company Ltd.) under 25°C conditions.

The chitin nanofiber and chitosan nanofiber to be used in the present invention are constituted of chitin and chitosan (i.e., chitinous substance) which are water-insoluble polysaccharides. Saccharides mean glycopolymers wherein not less than 10 single saccharides (e.g., triose, tetrose, pentose, hexsauce, heptose etc.) are polymerized.

The chitinous substance refers to one or more carbohydrates selected from the group consisting of chitin and chitosan. Major sugar units constituting chitin and chitosan are N-acetylglucosamine and glucosamine, respectively. Generally, chitin has a high N-acetylglucosamine content and is poorly soluble in acidic aqueous solution, and chitosan has a high glucosamine content and is soluble in acidic aqueous solution. The ratio of N-acetylglucosamine in the sugar unit constituting chitin is not less than 90%, and may be not less than 98%, or 100%. The ratio of N-acetylglucosamine in the sugar unit constituting chitosan is not more than 30%, and may be not more than 20%, or not more than 10%.

As the starting material of chitin, many biological resources such as shrimps, crabs, insect, shells, mushrooms and the like can be used. The chitin to be used in the present invention may be one having α-form crystal structure such as chitin derived from crab shell, shrimp shell and the like, or one having β-form crystal structure such as chitin derived from cuttlebones and the like. The test of crabs and shrimps is often regarded as industrial waste and preferable as a starting material since it is easily available and effectively used. On the other hand, it requires a protein removing step and a decalcification step to remove protein, minerals and the like contained as impurities. In the present invention, therefore, purified chitin that underwent a matrix removal treatment is preferably used. Purified chitin is commercially available. The starting material for the chitin nanofiber to be used in the present invention may be a chitin having any of the α type and β type crystal structures, but an α type chitin is preferable.

Chitosan can be produced by deacetylating chitin by boiling same in a concentrated alkali (e.g., concentrated NaOH aqueous solution or the like). As chitosan, commercially available ones may also be used.

Chitin nanofiber and chitosan nanofiber can be obtained by pulverizing (sometimes to be referred to as nanofiberizing or fibrillating) the aforementioned chitin and chitosan. While the pulverization method is not limited, a method affording a strong shear force such as a medium stirring mill, for example, a high-pressure homogenizer, a grinder (stone mill), a bead mill and the like is preferable for subdivision to the fiber diameter and fiber length meeting the object of the present invention. The mixture of chitin nanofiber and chitosan nanofiber to be used in the present invention may be prepared by mixing individually-fibrillated chitin nanofiber and chitosan nanofiber, or may be prepared by mixing chitin powder and chitosan powder and simultaneously fibrillating the mixture.

Of these, subdivision by a high-pressure homogenizer is preferable, and, for example, subdivision (pulverization) by the wet grinding method disclosed in JP-A-2005-270891 or JP-B-5232976 is desirable. Specifically, the starting material is pulverized by spraying a dispersion of a starting material from a pair of nozzles at a high-pressure and bombarding each other, and, for example, Star Burst system (high-pressure pulverization device manufactured by Sugino Machine Limited) or NanoVater (high-pressure pulverization device of yoshida kikai co., ltd.) is used therefor.

In the subdivision (pulverization) of a starting material by the aforementioned high-pressure homogenizer, the degree of subdivision and homogenization depends on the pressure in pumping into an ultrahigh-pressure chamber in a high-pressure homogenizer, and the number (treatment number) of passage through the ultrahigh-pressure chamber, and the concentration of the starting material in the water dispersion. The pumping pressure (treatment pressure) is not particularly limited and it is generally 50 - 250 MPa, preferably 100 - 200 MPa.

While the concentration of the starting material in a water dispersion during the subdividing treatment is not particularly limited, it is generally 0.1 mass % - 30 mass %, preferably 1 mass % - 10 mass %. While the treatment number of the subdivision (pulverization) is not particularly limited, it varies depending on the concentration of the starting material in the aforementioned water dispersion. When the concentration of the starting material is 0.1 - 1 mass %, the treatment number of 10 - 100 is sufficient for pulverization, but 1 - 10 mass % sometimes requires about 10 - 1000 times of treatment.

The viscosity of the water dispersion during the aforementioned subdivision treatment is not particularly limited. For example, in the case of α chitin, the viscosity of the water dispersion is within the range of 1 - 100 mPa·S, preferably 1 - 85 mPa·S (by tuning fork vibration type viscometer (SV-1A, A&D Company Ltd.) under 25°C conditions). In the case of chitosan, the viscosity of the water dispersion is within the range of 0.7 - 30 mPa·S, preferably 0.7 - 10 mPa·S (by tuning fork vibration type viscometer (SV-1A, A&D Company Ltd.) under 25°C conditions). The particle size of the chitin or chitosan in a water dispersion during a subdivision treatment is not particularly limited. For example, in the case of α chitin, the average particle size of α chitin in the water dispersion is within the range of 0.5 - 200 um, preferably 30 - 150 um (by laser diffraction/scattering type particle size distribution measurement apparatus LA-960 (Horiba, Ltd.)). In the case of chitosan, the average particle size of chitosan in the water dispersion is within the range of 0.5 - 300 µm, preferably 50 - 100 um (by laser diffraction/scattering type particle size distribution measurement apparatus LA-960 (Horiba, Ltd.)).

The preparation method of a nanofiber is described in WO 2015/111686 A1 and the like.

Polysaccharides may be added to the medium composition of the present invention. In the present specification, polysaccharides mean glycopolymers wherein not less than 10 single saccharides (e.g., triose, tetrose, pentose, hexsauce, heptose etc.) are polymerized.

Examples of the water-insoluble polysaccharides include, but are not limited to, celluloses such as cellulose, hemicellulose and the like; chitinous substances such as chitin, chitosan and the like, and the like.

Examples of the water-soluble polysaccharides include acidic polysaccharides having an anionic functional group. Examples of the acidic polysaccharides having an anionic functional group include, but are not limited to, polysaccharides having uronic acid (e.g., glucuronic acid, iduronic acid, galacturonic acid, mannuronic acid) in the structure; polysaccharides having sulfuric acid or phosphoric acid in the structure; polysaccharides having both structures and the like. More specifically, those constituted of one or more kinds selected from the group consisting of hyaluronic acid, gellan gum, deacylated gellan gum, rhamsan gum, diutan gum, xanthan gum, carrageenan, zanthan gum, hexuronic acid, fucoidan, pectin, pectic acid, pectinic acid, heparan sulfate, heparin, heparitin sulfate, keratosulfate, chondroitin sulfuric acid, dermatan sulfuric acid, rhamnan sulfate, alginic acid and salts thereof can be recited as examples.

Examples of the salts here include alkali metal salts such as lithium, sodium and potassium; alkaline earth metal salts such as calcium, barium and magnesium; salts such as aluminum, zinc, copper and iron; ammonium salts; quaternary ammonium salts such as tetraethylammonium, tetrabutylammonium, methyltributylammonium, cetyltrimethylammonium, benzylmethylhexyldecylammonium, choline and the like; salts with organic amine such as pyridine, triethylamine, diisopropyl amine, ethanolamine, diolamine, tromethamine, meglumine, procaine, chloroprocaine and the like; salt with amino acid such as glycine, alanine, valine and the like, and the like.

In one embodiment, as the polysaccharide, methylcellulose, deacylated gellan gum, alginic acid, xanthan gum, carrageenan, diutan gum, locust bean gum, tamarind seed gum, pectin, and carboxymethylcellulose or a salt thereof may be preferably used.

To achieve the desired effect in the medium composition of the present invention, the blending ratio of chitin nanofiber and chitosan nanofiber may be important. First, chitin nanofiber and chitosan nanofiber are blended in a ratio (weight) of chitin nanofiber:chitosan nanofiber=1:3 - 6. The obtained mixture of chitin nanofiber/chitosan nanofiber can be blended in a liquid medium such that the concentration of the total nanofibers (chitin nanofiber and chitosan nanofiber) contained in the medium composition is generally 0.0001 - 0.2%(w/v), preferably 0.0005 - 0.1%(w/v), further preferably 0.001 - 0.05%(w/v), particularly preferably 0.006 - 0.05%(w/v). Alternatively, the medium composition of the present invention may be prepared by separately adding the necessary amounts of chitin nanofiber and chitosan nanofiber to the liquid medium and stirring well. In one embodiment, the medium composition of the present invention satisfies the following conditions with respect to the concentration of nanofiber:
(1) the concentration of the total nanofibers (chitin nanofiber and chitosan nanofiber) contained in the medium composition is 0.0001 - 0.2%(w/v), and the weight ratio of chitin nanofiber:chitosan nanofiber contained in the medium composition is 1:3 - 6;
(2) the concentration of the total nanofibers (chitin nanofiber and chitosan nanofiber) contained in the medium composition is 0.0005 - 0.1%(w/v), and the weight ratio of chitin nanofiber:chitosan nanofiber contained in the medium composition is 1:3 - 6;
(3) the concentration of the total nanofibers (chitin nanofiber and chitosan nanofiber) contained in the medium composition is 0.001 - 0.05%(w/v), and the weight ratio of chitin nanofiber:chitosan nanofiber contained in the medium composition is 1:3 - 6; or
(4) the concentration of the total nanofibers (chitin nanofiber and chitosan nanofiber) contained in the medium composition is 0.006 - 0.05(w/v), and the weight ratio of chitin nanofiber:chitosan nanofiber contained in the medium composition is 1:3 - 6.

Athough not bound by theory, the fact that the blending ratio of chitin nanofiber and chitosan nanofiber is important for obtaining the desired effect of the present invention is considered to suggest that the total amount of acetylated glucosamine in chitin nanofiber and chitosan nanofiber is important. Therefore, also described herein is use of a nanofiber prepared from a single poly(1,4)-N-acetyl-β-D-glucosamine with optimized degree of acetylation and having an acetyl group number corresponding to the acetyl group number of the mixture of the two kinds of nanofibers (hereinafter sometimes referred to as "nanofiber with adjusted amount of N-acetylglucosamine" and the like), instead of using a mixture of two kinds of nanofibers, chitin nanofiber and chitosan nanofiber. Therefore, a medium for suspension culture of adherent cells which contains poly(1,4)-N-acetyl-β-D-glucosamine nanofiber having a specific acetylation degree is also described. The specific acetylation degree of such poly(1,4)-N-acetyl-β-D-glucosamine may be generally 5 - 70%, preferably 9 - 70%, more preferably 11 - 55%, further preferably 14 - 50%, further preferably 16 - 50%, particularly preferably 22 - 33%, though not limited to these as long as the desired effect is obtained.

The poly(1,4)-N-acetyl-β-D-glucosamine having the aforementioned specific acetylation degree can be produced by a method known per se. As one example, a method for producing poly(1,4)-N-acetyl-β-D-glucosamine having a desired acetylation degree, including deacetylating chitin with a relatively high rate of acetylation (e.g., 90% or more) by a boiling treatment in an alkali (e.g., concentrated NaOH aqueous solution, etc.) can be mentioned, but the method is not limited thereto.

The thus-obtained poly(1,4)-N-acetyl-β-D-glucosamine having a specific acetylation degree is refined (sometimes to be referred to as nanofiberized or fibrillated) by the aforementioned method, whereby poly(1,4)-N-acetyl-β-D-glucosamine nanofiber having a specific acetylation degree can be prepared.

Nanofiber with an adjusted amount of N-acetylglucosamine may be added to the liquid medium composition. The nanofiber with an adjusted amount of N-acetylglucosamine can be added to the liquid medium such that the concentration of the nanofiber contained in the liquid medium composition is generally 0.0001 - 0.2%(w/v), preferably 0.0005 - 0.1%(w/v), further preferably 0.001 - 0.1%(w/v), particularly preferably 0.006 - 0.06%(w/v). The concentration is not limited thereto as long as a desired effect can be obtained.

The medium contained in the medium composition of the present invention can be appropriately selected according to the kind and the like of the adherent cells to be used. For example, when used for the purpose of culture of mammalian adherent cells, a medium generally used for culturing mammalian cells can be used as a medium to be contained in the medium composition of the present invention. Examples of the medium for mammalian cells include Dulbecco's Modified Eagle's medium (DMEM), hamF12 medium (Ham's Nutrient Mixture F12), DMEM/F12 medium, McCoy's 5A medium, Eagle MEM medium (Eagle's Minimum Essential medium; EMEM), αMEM medium (alpha Modified Eagle's Minimum Essential medium; αMEM), MEM medium (Minimum Essential medium), RPMI1640 medium, Iscove's Modified Dulbecco's medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpan SFEM (manufactured by STEMCELL Technologies), StemlineII (manufactured by Sigma Aldrich), QBSF-60 (manufactured by Quality Biological), StemPro hESC SFM (manufactured by Invitrogen), mTeSR1 or 2 medium (manufactured by STEMCELL Technologies), Sf-900II (manufactured by Invitrogen), Opti-Pro (manufactured by Invitrogen), and the like.

Those of ordinary skill in the art can freely add, according to the object, sodium, potassium, calcium, magnesium, phosphorus, chlorine, various amino acids, various vitamins, antibiotic, serum, fatty acid, sugar and the like to the above-mentioned medium. For culture of mammalian cells, those of ordinary skill in the art can also add, according to the object, one or more kinds of other chemical components and biogenic substances in combination. Examples of the components to be added to a medium for mammalian cells include fetal bovine serum, human serum, horse serum, insulin, transferrin, lactoferrin, cholesterol, ethanolamine, sodium selenite, monothioglycerol, 2-mercaptoethanol, bovine serum albumin, sodium pyruvate, polyethylene glycol, various vitamins, various amino acids, agar, agarose, collagen, methylcellulose, various cytokines, various hormones, various proliferation factors, various extracellular matrices, various cell adhesion molecules and the like. Examples of the cytokine to be added to a medium include, but are not limited to, interleukin-1 (IL-1), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), interleukin-13 (IL-13), interleukin-14 (IL-14), interleukin-15 (IL-15), interleukin-18 (IL-18), interleukin-21 (IL-21), interferon-α (IFN-α), interferon-β (IFN-β), interferon-γ (IFN-γ), granulocyte colony stimulating factor (G-CSF), monocyte colony stimulating factor (M-CSF), granulocytemacrophage colony stimulating factor (GM-CSF), stem cell factor (SCF), flk2/flt3 ligand (FL), leukemia cell inhibitory factor (LIF), oncostatin M (OM), erythropoietin (EPO), thrombopoietin (TPO) and the like.

Examples of the hormone to be added to a medium include, but are not limited to, melatonin, serotonin, thyroxine, triiodothyronine, epinephrine, norepinephrine, dopamine, anti-Mullerian hormone, adiponectin, adrenocorticotropic hormone, angiotensinogen and angiotensin, antidiuretic hormone, atrial natriuretic peptide, calcitonin, cholecystokinin, corticotropin release hormone, erythropoietin, follicle stimulating hormone, gastrin, ghrelin, glucagon, gonadotropin release hormone, growth hormone release hormone, human chorionic gonadotropin, human placental lactogen, growth hormone, inhibin, insulin, insulin-like growth factor, leptin, luteinizing hormone, melanocyte stimulating hormone, oxytocin, parathyroid hormone, prolactin, secretin, somatostatin, thrombopoietin, thyroidstimulating hormone, thyrotropin releasing hormone, cortisol, aldosterone, testosterone, dehydroepiandrosterone, androstenedione, dihydrotestosterone, estradiol, estrone, estriol, progesterone, calcitriol, calcidiol, prostaglandin, leukotriene, prostacyclin, thromboxane, prolactin releasing hormone, lipotropin, brain natriuretic peptide, neuropeptide Y, histamine, endothelin, pancreas polypeptide, rennin and enkephalin.

Examples of the growth factor to be added to a medium include, but are not limited to, transforming growth factor-α (TGF-α), transforming growth factor-β (TGF-β), macrophage inflammatory protein-1α (MIP-1α), epithelial cell growth factor (EGF), fibroblast growth factor-1, 2, 3, 4, 5, 6, 7, 8 or 9 (FGF-1, 2, 3, 4, 5, 6, 7, 8, 9), nerve cell growth factor (NGF) hepatocyte growth factor (HGF), leukemia inhibitory factor (LIF), protease nexin I, protease nexin II, plateletderived growth factor (PDGF), choline vasoactive differentiation factor (CDF), chemokine, Notch ligand (Delta1 and the like), Wnt protein, angiopoietin-like protein 2, 3, 5 or 7 (Angpt2, 3, 5, 7), insulin like growth factor (IGF), insulin-like growth factor binding protein (IGFBP), Pleiotrophin and the like.

In addition, these cytokines and growth factors having amino acid sequences artificially altered by gene recombinant techniques can also be added. Examples thereof include IL-6/soluble IL-6 receptor complex, Hyper IL-6 (fusion protein of IL-6 and soluble IL-6 receptor) and the like.

Examples of the various extracellular matrices and various cell adhesion molecules include collagen I to XIX, fibronectin, laminin-1 to 12, nitrogen, tenascin, thrombospondin, von Willebrand factor, osteopontin, fibrinogen, various elastins, various proteoglycans, various cadherins, desmocolin, desmoglein, various integrins, E-selectin, P-selectin, L-selectin, immunoglobulin superfamily, Matrigel, poly-D-lysine, poly-L-lysine, chitin, chitosan, sepharose, hyaluronic acid, alginate gel, various hydrogels, cleavage fragments thereof and the like.

Examples of the antibiotic to be added to a medium include Sulfonamides and preparations, penicillin, phenethicillin, methicillin, oxacillin, cloxacillin, dicloxacillin, flucloxacillin, nafcillin, ampicillin, penicillin, amoxicillin, ciclacillin, carbenicillin, ticarcillin, piperacillin, azlocillin, mezlocillin, mecillinam, andinocillin, cephalosporin and a derivative thereof, oxolinic acid, amifloxacin, temafloxacin, nalidixic acid, Piromidic acid, ciprofloxacin, cinoxacin, norfloxacin, perfloxacin, Rosaxacin, ofloxacin, enoxacin, pipemidic acid, sulbactam, clavulanic acid, β-bromopenisillanic acid, β-chloropenisillanic acid, 6-acetylmethylene-penisillanic acid, cephoxazole, sultampicillin, adinoshirin and sulbactam formaldehyde hudrate ester, tazobactam, aztreonam, sulfazethin, isosulfazethin, norcardicin, phenylacetamidophosphonic acid methyl, Chlortetracycline, oxytetracycline, tetracycline, demeclocycline, doxycycline, methacycline, and minocycline.

As described above, a serum and/or a serum replacement may be added to the medium composition of the present invention. Using the medium composition of the present invention, cells can be maintained and/or expanded even when the concentration of the serum and/or serum replacement in the medium is lower than the concentration used generally (e.g., 10% by weight). The concentration of the serum to be added to the medium composition of the present invention can be appropriately set depending on the kind of the cell, culture conditions, purpose of culture and the like. In one embodiment, the concentration of the serum (and/or serum replacement) of the medium composition of the present invention may be set to not more than 15 wt%, not more than 10 wt%, not more than 9 wt%, not more than 8 wt%, not more than 7 wt%, not more than 6 wt%, not more than 5 wt%, not more than 4 wt%, not more than 3 wt%, not more than 2 wt%, not more than 1 wt%, not more than 0.9 wt%, not more than 0.8 wt%, not more than 0.7 wt%, not more than 0.6 wt%, not more than 0.5 wt%, not more than 0.4 wt%, or not more than 0.3 wt%. The serum concentration may be constant during the culture period, or may be increased or decreased as necessary at the time of medium change or the like.

In one embodiment, when the purpose of cell culture is production of a cell secretion product for regenerative medicine, it is sometimes preferable to set a low serum concentration of the medium composition of the present invention in order to reduce the serum risk. In such embodiment, the concentration of the serum in the medium composition of the present invention can be set to, for example, not more than 2 wt%, not more than 1 wt%, not more than 0.9 wt%, not more than 0.8 wt%, not more than 0.7 wt%, not more than 0.6 wt%, not more than 0.5 wt%, not more than 0.4 wt%, or not more than 0.3 wt%.

In another embodiment, when the number of cells to be subjected to -expansion culture is maximized under low serum concentrations, the serum concentration may be set to generally 0.05 - 2.0 wt%, preferably 0.1 - 2.0 wt%, further preferably 0.2 - 2.0 wt%, particularly preferably 0.2 - 1.0 wt%.

In another embodiment, the range of serum concentration when maximizing the yield of a cell secretion product may be set to generally 0.05 - 2.0 wt%, preferably 0.1 - 1.5 wt%, further preferably 0.2 - 0.9 wt%, particularly preferably 0.4 - 1.0 wt%.

The period of maintenance culture and/or expansion culture of cells in a low serum medium can be appropriately set according to the kind of the cell, culture conditions, or purpose of culture. For example, it can be set to not less than one day, not less than 3 days, not less than 5 days, not less than 7 days, not less than 9 days, not less than 11 days, not less than 13 days, not less than 15 days, not less than 20 days, not less than 25 days, not less than 30 days, not less than 40 days, not less than 50 days, not less than 60 days, not less than 80 days, or not less than 100 days.

In one embodiment, the culture period can be set to 1 - 200 days, 1 - 100 days, 1 - 80 days, 1 - 60 days, 1 - 50 days, 1 - 40 days, 1 - 30 days, 1 - 25 days, 1 - 20 days, 1 - 15 days, 1 - 13 days, 1 - 11 days, 1 - 9 days, 1 - 7 days, 1 - 5 days, or 1 - 3 days. In a more preferable embodiment, the culture period can be set to preferably 10 - 60 days, more preferably 15 - 50 days, further preferably 20 days - 50 days, particularly preferably 25 days - 40 days.

The above-mentioned medium composition of the present invention can be produced by mixing the above-mentioned chitin nanofibers and chitosan nanofibers with an appropriate liquid medium such that a concentration at which the adherent cells can be suspended (preferably suspension standing) can be achieved, when the adherent cells are subjected to suspension culture by using the medium composition.

In a preferred embodiment, the medium composition of the present invention can be prepared by mixing a dispersion of the above-mentioned chitin nanofiber and chitosan nanofiber in an aqueous solvent with a liquid medium. The dispersion may be sterilized (autoclave, gamma sterilization etc.). Alternatively, the dispersion and a liquid medium (aqueous solution as medium) prepared by dissolving the powder medium in water may be mixed, and used after sterilization. The dispersion and the liquid medium may be sterilized separately before mixing. Examples of the aqueous solvent include, but are not limited to, water, dimethyl sulfoxide (DMSO) and the like. As the aqueous solvent, water is preferable. The aqueous solvent may contain appropriate buffering agents and salts. The above-mentioned nanofiber dispersion is useful as a medium additive for preparing the medium composition of the present invention.

While the mixing ratio is not particularly limited, nanofiber dispersion:liquid medium (aqueous solution as medium) (volume ratio) is generally 1:99 - 99:1, preferably 10:90 - 90:10, more preferably, 20:80 - 80:20.

Suspending of cells in the present invention refers to a state where cells do not adhere to a culture container (nonadhesive), and whether the cell forms sediment is not questioned. Furthermore, in the present invention, when the cells are cultured, the state where the cells and/or tissues are dispersed and suspended in the liquid medium composition in the absence of a pressure on or vibration of the liquid medium composition from the outside or shaking, rotating operation and the like in the composition is referred to as "suspension standing", and cultivation of the cells and/or tissues in such condition is referred to as "suspension standing culture". In the "suspension standing", the period of suspending includes at least 5 min, preferably, not less than 1 hr, not less than 24 hr, not less than 48 hr, not less than 6 days, not less than 21 days, though the period is not limited thereto as long as the suspended state is maintained.

In a preferable embodiment, the medium composition of the present invention permits suspension standing of cells at least on one point in the temperature range (e.g., 0 - 40°C) capable of culturing cells. The medium composition of the present invention permits suspension standing of cells at least on one point in the temperature range of preferably 25 - 37°C, most preferably 37°C.

Suspension culture of adherent cells can be performed by culturing adherent cells while being attached to a chitin nanofiber and chitosan nanofiber. The suspension culture can be performed by culturing adherent cells in the above-mentioned medium composition of the present invention. A chitin nanofiber and chitosan nanofiber show an effect of suspending the cells attached to the nanofibers in the medium (preferably effect of suspension standing). In the medium composition of the present invention, since chitin nanofiber and chitosan nanofiber are dispersed without dissolving or attaching to a culture container, when adherent cells are cultured in the medium composition, the adherent cells attach to the nanofibers and are suspended in the medium composition. By the suspending effect, a more increased amount of the number of cells per a given volume can be cultivated as compared with monolayer culture. In conventional suspension culture accompanying rotation or shaking operation, the proliferation rate and recovery rate of the cells may become low, or the function of the cell may be impaired since a shear force acts on the cells. Using the medium composition of the present invention, the cells can be cultured in a dispersion state without requiring an operation such as shaking and the like. Thus, easy suspension culture of a large amount of the object adherent cells without loss of the cell function can be expected. In addition, when cells are suspension cultured in a conventional medium containing a gel substrate, observation and recovery of the cells are sometimes difficult, and the function thereof is sometimes impaired during recovery. However, using the medium composition of the present invention, the cells under suspension culture are expected to be observed and recovered without impairing the function thereof. In addition, a conventional medium containing a gel substrate sometimes shows high viscosity that makes it difficult to exchange the medium. However, since the medium composition of the present invention has low viscosity, it is expected to be exchanged easily with a pipette, pump and the like.

When suspension culture of adherent cells is performed using chitin nanofiber and chitosan nanofiber, adherent cells prepared separately are added to the culture composition of the present invention and mixed uniformly. In this case, the mixing method is not particularly limited and, for example, manual mixing using pipetting and the like, mixing using instrument such as stirrer, vortex mixer, microplate mixer, shaking machine and the like can be mentioned. After mixing, the obtained cell suspension may be cultured while being stood still, or cultured with rotation, shaking or stirring as necessary. The rotating speed and frequency can be appropriately set according to the object of those of ordinary skill in the art. For example, adherent cells are recovered from the passage culture, dispersed to a single cell or close thereto using an appropriate cell dissociation solution, the dispersed adherent cells are suspended in the medium composition of the present invention, and this is subjected to suspension culture (preferably, suspension standing culture).

The temperature when cells are cultivated is generally 25 to 39°C (e.g., 37°C), preferably 33 to 39°C, for animal cells. The CO₂ concentration is generally 4 to 10% by volume in the culture atmosphere, and 4 to 6% volume is preferable. The culture period may be set as appropriately according to the object of the culture.

When adherent cells are cultivated in the medium composition of the present invention, culture vessels generally used for cell culture such as schale, flask, plastic bag, Teflon (registered trade mark) bag, dish, schale, dish for tissue culture, multidish, microplate, microwell plate, multiplate, multiwell plate, chamber slide, tube, tray, culture bag, roller bottle and the like can be used for cultivation. These culture containers are desirably low cell -adhesive so that the adherent cells attached to a nanofiber will not adhere to the culture container. As a low cell -adhesive culture vessel, a culture vessel having a surface not artificially treated to improve adhesiveness to cells (e.g., coating treatment with extracellular matrix and the like), or a culture vessel having a surface artificially treated to reduce adhesiveness to cells can be used.

When the medium needs to be exchanged, the cells are separated by centrifugation or filtration treatment, and a fresh medium or the medium composition of the present invention can be added of the cells. Alternatively, the cells are appropriately concentrated by centrifugation or filtration treatment, and a fresh medium or the medium composition of the present invention can be added to the concentrated liquid. For example, unlimitatively, the gravitational acceleration (G) of centrifugation is 100G to 400G, and the size of the pore of the filter used for the filtration treatment is 10 µm to 100 µm.

The adherent cells can also be cultured by automatically conducting cell seeding, medium exchange, cell image obtainment, and recovery of cultured cells, under a mechanical control and under a closed environment while controlling pH, temperature, oxygen concentration and the like and using a bioreactor and an automatic incubator capable of high-density culture.

Since adherent cells are efficiently proliferated when they are subjected to suspension culture while being attached to chitin nanofiber, and chitosan nanofiber, the suspension culture is superior as a maintenance or proliferation method of the adherent cells. When adherent cells are subjected to suspension culture while being attached to chitin nanofiber, and chitosan nanofiber, they are dispersed while spreading three-dimensionally, without adhering to a culture container or without being locally present only on the bottom surface of the culture container, whereby the proliferation is promoted. As a result, the proliferated cells are connected like cluster of grapes on the nanofiber. This proliferation-promoting effect only requires presence of nanofibers at a concentration sufficient for suspending adherent cells (i.e., avoiding adhesion of adherent cells to culture container) in the medium composition, and capability of suspension standing (i.e., cells being uniformly dispersed and in a suspended state in liquid medium composition, without the presence of pressure, trembling, shaking, rotating operation and the like from the outside) is not essential.

When adherent cells are subjected to suspension culture while being attached to chitin nanofiber, and chitosan nanofiber to proliferate the adherent cells, a medium permitting proliferation of the adherent cells while maintaining the character thereof is used as the medium contained in the medium composition of the present invention to be used for the suspension culture. Those of ordinary skill in the art can appropriately select the medium according to the kind of the adherent cells.

In one embodiment, mesenchymal stem cell are maintained or proliferated by performing suspension culture of the cells while they are attached to chitin nanofiber, and chitosan nanofiber. By the suspension culture, mesenchymal stem cell can be proliferated while maintaining the differentiation potency and homing/chemotactic activity thereof. Therefore, using the medium composition of the present invention, high-quality stem cells can be prepared. Whether the stem cell maintains properties such as differentiation potency, chemotactic activity and the like can be determined by a method known per se. Briefly, it can be easily judged by determining, at mRNA level and/or protein level, the expression levels of cell markers relating to undifferentiated state in stem cells (e.g., OCT4 gene, SOX2 gene, NANOG gene, etc.) and cell markers relating to chemotacticity (e.g., CXCR4 gene, etc.).

When adherent cells are subjected to suspension culture while being attached to chitin nanofiber, and chitosan nanofiber, the adherent cells can be passaged by simply adding a fresh medium or the medium composition of the present invention to the suspension culture, or just adding a culture after culture entirely or partly to a fresh medium or the medium composition of the present invention, without a detaching operation of the cells from a culture container. Therefore, using the passage culture method of the present invention, adherent cells can be passaged without a detaching operation of the cells from a culture container. Using this passage culture method, moreover, the culture scale of adherent cells can be expanded without a detaching operation of the cells from a culture container. As a detaching operation of the cells from a culture container, a treatment with a chelating agent (e.g., EDTA) and/or protease (e.g., trypsin, collagenase) can be mentioned. The above-mentioned passage culture method is advantageous for passage culture of adherent cells highly sensitive to a detaching operation of the cells from a culture container (e.g., adherent cell with viability decreased by detaching operation, adherent cell with character susceptible to change by detaching operation). Examples of the adherent cells highly sensitive to a detaching operation of the cells from a culture container include mesenchymal stem cell.

The adherent cells proliferated using the medium composition of the present invention can be obtained by decomposing chitin nanofiber and chitosan nanofiber by using one or more kinds of degrading enzymes such as chitinase, chitobiase, chitosanase, β-1,3-glucanase and the like, and detaching and recovering the adherent cells from the nanofibers. Yatalase (Takara Bio Inc.) is a mixture of chitinase, chitobiase, chitosanase and β-1,3-glucanase, and can be preferably used as a degrading enzyme of chitin or chitosan.

For example, a degrading enzyme of chitin and chitosan is added to a suspension of adherent cells attached to chitin nanofiber and chitosan nanofiber and the mixture is incubated for a time sufficient for the detachment of the adherent cells. The temperature of incubation by the degrading enzyme of chitin and chitosan is generally 20°C - 37°C. The incubation time varies depending on the kind of the enzyme and the like and is generally 5 - 60 min.

When the nanofiber is degraded and the adherent cells are detached from the nanofiber, the detached adherent cells can be recovered by subjecting the suspension to centrifugation.

Since the damage on the thus-recovered adherent cells is minimized, the cells can be preferably used for functional analysis, transplantation and the like.

### 2. Method for culturing adherent cells

The present invention also provides a method for culturing a mesenchymal steo cell, including a step of suspension culturing the mesenchymal stem cell in a medium composition containing (1) a chitin nanofiber; and (2) a chitosan nanofiber wherein the ratio of the chitin nanofiber and the chitosan nanofiber is chitin nanofiber:chitosan nanofiber=1:3 - 6;
wherein the ratio of N-acetylglucosamine in the sugar units constituting chitin is not less than 90%; and
wherein the ratio of N-acetylglucosamine in the sugar units constituting chitosan is not more than 30%
   (hereinafter sometimes referred to as "the culture method of the present invention").

The culture method of the present invention is characterized in that adherent cells are cultured using the aforementioned medium composition of the present invention. Therefore, the medium composition, adherent cell, culture conditions and the like in the culture method of the present invention are the same as those described in the medium composition of the present invention.

### 3. Method for producing cell secretion product

The present invention also provides a method for producing a cell secretion product, including a step of suspension culturing the mesenchymal stem cell in a medium composition containing (1) a chitin nanofiber; and (2) a chitosan nanofiber wherein the ratio of the chitin nanofiber and the chitosan nanofiber is chitin nanofiber:chitosan nanofiber=1:3 - 6;
wherein the ratio of N-acetylglucosamine in the sugar units constituting chitin is not less than 90%; and
wherein the ratio of N-acetylglucosamine in the sugar units constituting chitosan is not more than 30%
   (hereinafter sometimes referred to as "the production method of the present invention").

The production method of the present invention is characterized in that adherent cells are cultured using the aforementioned medium composition of the present invention. Therefore, the medium composition, adherent cell, culture conditions and the like in the culture method of the present invention are the same as those described in the medium composition of the present invention.

The adherent cell is a mesenchymal stem cell. Such mesenchymal stem cell may be derived from any of bone marrow, adipocyte, umbilical cord, pulp and the like.

The cell secretion product in the production method of the present invention includes any substances secreted by cells such as low-molecular-weight compounds, proteins, nucleic acids (miRNA, mRNA, etc.), cell secretion vesicles (see below), and the like.

Examples of the cell secretion product produced by the production method of the present invention include, but are not limited to, prostaglandin E2 (PGE2), basic fibroblast growth factor (bFGF), Tumor necrosis factor-stimulated gene-6 (TSG-6), vascular endothelium growth factor (VEGF), indoleamine-2,3-dioxygenase (IDO), transforming growth factor β (TGF-β), hepatocyte growth factor (HGF), insulin-like growth factor (IGF), brain-derived neurotrophic factor (BDNF), nerve growth factor (NGF), angiopoietin-1 and the like.

In the present specification, the cell secretion vesicle typically means a vesicle released from a cell and having a size that can be confirmed by an electron microscope. The size of such cell secretion vesicle may be 1 - 1,000 nm, 10 - 500 nm, or 30 - 200 nm in average particle size. The average particle size here is an average of the diameters of respective particles measured by a dynamic light scattering method or an electron microscope. Such cell secretion vesicle may consist of a lipid bilayer that surrounds the biomolecule.

Specific examples of the cell secretion vesicle include, but are not limited to, membrane particle, membrane vesicle, microvesicle, nanovesicle, microvesicle (average particle size 30 - 1,000 nm), exosome-like vesicle, exosome (average particle size 30 - 200 nm), ectosome-like vesicle, ectosome, exovesicle and the like. In one preferable embodiment of the production method of the present invention, the cell secretion vesicle may be an exosome.

In one embodiment of the production method of the present invention, a compound for initiating and/or promoting the production of cell secretion products may be added. As the compound for initiating and/or promoting the production of cell secretion products, a known compound may be selected according to the cell secretion product to be produced. Examples of the compound include, but are not limited to, TNF-α, IFN-γ, and interleukin-1β (IL-1β). In one embodiment, when the cell secretion product is PGE2 or TSG-6, it may be preferable to add TNF-α to the medium composition. Also, when the cell secretion product is PGE2 or IDO, it may be preferable to add IFN-γ to the medium composition. The concentration of the compound added to the medium composition for initiating and/or promoting the production of the cell secretion product is not particularly limited as long as the desired effect can be obtained, and may be appropriately determined based on the combination of the cell secretion product to be produced and the compound, and the like.

In another embodiment of the production method of the present invention, the cells and culture conditions used in the production method of the present invention may be appropriately optimized to increase the production amount of cell secretion products. As one embodiment, it may be preferable to use adherent cells (i.e., mesenchymal stem cells) exposed to low oxygen conditions in the production method of the present invention (see J Cell Mol Med. 2018 Mar; 22(3):1428-1442). Any technique known per se may be used for such optimization of cells and/or culture conditions.

The present invention is explained in more detail in the following by referring to Examples; however, the present invention is not limited in any manner by these Examples.

### [Example]

### (Experimental Example 1: Proliferation of human adipose tissue-derived mesenchymal stem cells by 3D culture using mixture of chitin nanofiber and chitosan nanofiber)

α chitin nanofiber (proportion of N-acetylglucosamine: not less than 95%) or chitosan nanofiber (proportion of N-acetylglucosamine: not more than 20%) (biomass nanofiber BiNFi-S 2 mass %, Sugino Machine Limited) prepared according to the production method disclosed in WO 2015/111686 was suspended in ultrapure water (Milli-Q water) to 1% (w/v), dispersed by inversion mixing, and the aqueous solutions were autoclave sterilized at 121°C for 20 min. The α chitin nanofibers used were α chitin nanofiber nanofiberized under condition of 200 MPa, pass 5 times (sample 1). The chitosan nanofibers were chitosan nanofibers nanofibrerized under condition of 200 MPa, pass 5 times (sample 2). Furthermore, sample 1 and sample 2 were mixed such that the ratio (volume) of sample 1 and sample 2 was 50%:50% (sample 3), 33%:67% (sample 4), 25%:75% (sample 5), 20%:80% (sample 6), 15%:85% (sample 7), 10%:90% (sample 8), 5%:95% (sample 9), and 1%:99% (sample 10) to give chitin nanofiber/chitosan nanofiber mixtures (samples 3 - 10).

Samples include samples with chitin nanofiber/chitosan nanofiber ratios outside the scope of the present invention for comparative purposes.

Medium compositions were prepared by adding samples 1 - 10 obtained above to a mesenchymal stem cell proliferation medium (C-28009, manufactured by Takara Bio Inc.), which is a serum medium, at various final concentrations (final concentration: 0.0006% (w/v), 0.002% (w/v), 0.006% (w/v), 0.02% (w/v)), and a non-addition medium composition (sample 11) which was free of the above-mentioned substrate was prepared. Successively, the cultured human adipose-derived mesenchymal stem cells (C-12977, manufactured by Takara Bio Inc.) were suspended in each of the above-mentioned medium compositions at 13333 cells/mL, and seeded in a 96-well flat bottom ultra low attachment surface microplate (manufactured by Corning Incorporated, #3474) at 150 µL/well. The cells were cultured in a CO₂ incubator (37°C, 5% CO₂) in a static state for 10 days. An ATP reagent (150 µL, CellTiter-Glo^{™} Luminescent Cell Viability Assay, manufactured by Promega) was added to the culture medium on days 4, 8 after seeding and suspended and the suspension was stood for about 10 min at room temperature. The luminescence intensity (RLU value) was measured by FlexStation3 (manufactured by Molecular Devices) and the luminescence value of the medium alone was subtracted to calculate the number (mean of 3 samples) of viable cells.

As a result, when human adipose-derived mesenchymal stem cells were cultured in a medium composition containing a mixture of α chitin nanofiber and chitosan nanofiber in a 96-well flat bottom ultra low attachment surface microplate, and not less than 5 wt% of the total amount of α chitin nanofiber and chitosan nanofiber contained in the medium composition was α chitin nanofiber, a cell proliferation promoting effect was exhibited. When not less than 25 wt% of the total amount of α chitin nanofiber and chitosan nanofiber contained in the medium composition was α chitin nanofiber, an equivalent level of cell proliferation promoting effect was shown as compared with addition of α chitin nanofiber alone. The RLU values (ATP measurement, luminescence intensity) in respective cultures are shown in Table 1, Table 2 and Table 3.

**[Table 1]**

| | addition concentration (% (w/v)) | α chitin nanofiber (% (w/v)) | chitosan nanofiber (% (w/v)) | day 4 (cell number) | day 8 (cell number) |
|---|---|---|---|---|---|
| no addition (sample 11) | 0 | 0 | 0 | 4497 | 4645 |
| sample 1 | 0.0006 | 0.0006 | 0 | 7199 | 11322 |
| sample 1 | 0.002 | 0.002 | 0 | 8576 | 17237 |
| sample 1 | 0.006 | 0.006 | 0 | 8621 | 17967 |
| sample 1 | 0.02 | 0.02 | 0 | 8253 | 16248 |
| sample 3 | 0.0006 | 0.0003 | 0.0003 | 7826 | 11783 |
| sample 3 | 0.002 | 0.001 | 0.001 | 8840 | 15982 |
| sample 3 | 0.006 | 0.003 | 0.003 | 9106 | 18086 |
| sample 3 | 0.02 | 0.01 | 0.01 | 8965 | 14807 |
| sample 4 | 0.0006 | 0.0002 | 0.0004 | 8521 | 13091 |
| sample 4 | 0.002 | 0.00066 | 0.00134 | 8128 | 15131 |
| sample 4 | 0.006 | 0.002 | 0.004 | 9281 | 16592 |
| sample 4 | 0.02 | 0.0066 | 0.0134 | 8681 | 15563 |
| sample 5 | 0.0006 | 0.00015 | 0.00045 | 8091 | 11637 |
| sample 5 | 0.002 | 0.0005 | 0.0015 | 8305 | 15511 |
| sample 5 | 0.006 | 0.0015 | 0.0045 | 8660 | 15877 |
| sample 5 | 0.02 | 0.005 | 0.015 | 8831 | 14308 |

**[Table 2]**

| | addition concentration (%(w/v)) | α chitin nanofiber (%(w/v)) | chitosan nanofiber (% (w/v)) | day 4 (cell number) | day 8 (cell number) |
|---|---|---|---|---|---|
| no addition (sample 11) | 0 | 0 | 0 | 5310 | 5172 |
| sample 1 | 0.0006 | 0.0006 | 0 | 8323 | 11133 |
| sample 1 | 0.002 | 0.002 | 0 | 8971 | 17892 |
| sample 1 | 0.006 | 0.006 | 0 | 9431 | 18533 |
| sample 1 | 0.02 | 0.02 | 0 | 9033 | 16561 |
| sample 6 | 0.0006 | 0.00012 | 0.00048 | 8197 | 10659 |
| sample 6 | 0.002 | 0.0004 | 0.0016 | 8565 | 13953 |
| sample 6 | 0.006 | 0.0012 | 0.0048 | 9511 | 16357 |
| sample 6 | 0.02 | 0.004 | 0.016 | 9191 | 14821 |
| sample 7 | 0.0006 | 0.00009 | 0.00051 | 8233 | 11726 |
| sample 7 | 0.002 | 0.0003 | 0.0017 | 9083 | 13067 |
| sample 7 | 0.006 | 0.0009 | 0.0051 | 8994 | 15627 |
| sample 7 | 0.02 | 0.003 | 0.017 | 9273 | 13158 |
| sample 8 | 0.0006 | 0.00006 | 0.00054 | 8487 | 12210 |
| sample 8 | 0.002 | 0.0002 | 0.0018 | 8415 | 12155 |
| sample 8 | 0.006 | 0.0006 | 0.0054 | 9114 | 14262 |
| sample 8 | 0.02 | 0.002 | 0.018 | 8838 | 12877 |

**[Table 3]**

| | addition concentration (% (w/v)) | α chitin nanofiber (% (w/v)) | chitosan nanofiber (% (w/v)) | day 4 (cell number) | day 8 (cell number) |
|---|---|---|---|---|---|
| no addition (sample 11) | 0 | 0 | 0 | 4217 | 5202 |
| sample 1 | 0.0006 | 0.0006 | 0 | 8067 | 14232 |
| sample 1 | 0.002 | 0.002 | 0 | 9146 | 18102 |
| sample 1 | 0.006 | 0.006 | 0 | 8790 | 17444 |
| sample 1 | 0.02 | 0.02 | 0 | 8456 | 13679 |
| sample 9 | 0.0006 | 0.00003 | 0.00057 | 7451 | 9347 |
| sample 9 | 0.002 | 0.0001 | 0.0019 | 7528 | 10821 |
| sample 9 | 0.006 | 0.0003 | 0.0057 | 7591 | 11740 |
| sample 9 | 0.02 | 0.001 | 0.019 | 8106 | 11778 |
| sample 10 | 0.0006 | 0.000006 | 0.000594 | 7587 | 8802 |
| sample 10 | 0.002 | 0.00002 | 0.0018 | 7494 | 7568 |
| sample 10 | 0.006 | 0.00006 | 0.00594 | 7198 | 8508 |
| sample 10 | 0.02 | 0.0002 | 0.018 | 7169 | 9057 |
| sample 2 | 0.0006 | 0 | 0.0006 | 7144 | 8280 |
| sample 2 | 0.002 | 0 | 0.002 | 6953 | 7554 |
| sample 2 | 0.006 | 0 | 0.006 | 7247 | 8666 |
| sample 2 | 0.02 | 0 | 0.02 | 7018 | 8246 |

### (Experimental Example 2: Proliferation of human bone marrow tissue-derived mesenchymal stem cells by 3D culture using mixture of chitin nanofiber and chitosan nanofiber)

α chitin nanofiber or chitosan nanofiber (biomass nanofiber BiNFi-S 2 mass %, Sugino Machine Limited) prepared according to WO 2015/111686 was suspended in ultrapure water (Milli-Q water) to 1% (w/v), dispersed by inversion mixing, and the aqueous solutions were autoclave sterilized at 121°C for 20 min. The α chitin nanofibers used were α chitin nanofiber nanofiberized under condition of 200 MPa, pass 5 times (sample 1). The chitosan nanofibers were chitosan nanofibers nanofiberized under condition of 200 MPa, pass 5 times (sample 2). Furthermore, sample 1 and sample 2 were mixed such that the ratio (volume) of sample 1 and sample 2 was 50%:50% (sample 3), 33%:67% (sample 4), 20%:80% (sample 6) to give chitin nanofiber/chitosan nanofiber mixtures (samples 3, 4, 6). Samples include samples with chitin nanofiber/chitosan nanofiber ratios outside the scope of the present invention for comparative purposes.

Medium compositions were prepared by adding samples 1 - 4, 6 obtained above to a mesenchymal stem cell proliferation medium (C-28009, manufactured by Takara Bio Inc.), which is a serum medium, at various final concentrations (final concentration: 0.0006% (w/v), 0.002% (w/v), 0.006% (w/v), 0.02% (w/v)), and a non-addition medium composition (sample 11) which was free of the above-mentioned substrate was prepared. Successively, the cultured human bone marrow-derived mesenchymal stem cells (C-12977, manufactured by Takara Bio Inc.) were suspended in each of the above-mentioned medium compositions at 13333 cells/mL, and seeded in a 96-well flat bottom ultra low attachment surface microplate (manufactured by Corning Incorporated, #3474) at 150 µL/well. The cells were cultured in a CO₂ incubator (37°C, 5% CO₂) in a static state for 10 days. An ATP reagent (150 µL, CellTiter-Glo^{™} Luminescent Cell Viability Assay, manufactured by Promega) was added to the culture medium on day 7 after seeding and suspended and the mixture was stood for about 10 min at room temperature. The luminescence intensity (RLU value) was measured by FlexStation3 (manufactured by Molecular Devices) and the luminescence value of the medium alone was subtracted to calculate the number (mean of 3 samples) of viable cells.

As a result, when human bone marrow-derived mesenchymal stem cells were cultured in a medium composition containing a mixture of α chitin nanofiber and chitosan nanofiber in a 96-well flat bottom ultra low attachment surface microplate, and not less than 20 wt% of the total amount of α chitin nanofiber and chitosan nanofiber contained in the medium composition was α chitin nanofiber, an equivalent cell proliferation promoting effect was shown as compared with addition of α chitin nanofiber alone (i.e., sample 1). The RLU values (ATP measurement, luminescence intensity) in respective cultures are shown in Table 4.

**[Table 4]**

| | addition concentration (% (w/v)) | α chitin nanofiber (% (w/v)) | chitosan nanofiber (% (w/v)) | day 7 (cell number) |
|---|---|---|---|---|
| no addition (sample 11) | 0 | 0 | 0 | 2147 |
| sample 1 | 0.0006 | 0.0006 | 0 | 5808 |
| sample 1 | 0.002 | 0.002 | 0 | 8421 |
| sample 1 | 0.006 | 0.006 | 0 | 7826 |
| sample 1 | 0.02 | 0.02 | 0 | 5658 |
| sample 3 | 0.0006 | 0.0003 | 0.0003 | 5399 |
| sample 3 | 0.002 | 0.001 | 0.001 | 7566 |
| sample 3 | 0.006 | 0.003 | 0.003 | 8771 |
| sample 3 | 0.02 | 0.01 | 0.01 | 7249 |
| sample 4 | 0.0006 | 0.0002 | 0.0004 | 5218 |
| sample 4 | 0.002 | 0.00066 | 0.00134 | 7169 |
| sample 4 | 0.006 | 0.002 | 0.004 | 8174 |
| sample 4 | 0.02 | 0.0066 | 0.0134 | 6833 |
| sample 6 | 0.0006 | 0.00012 | 0.00048 | 5074 |
| sample 6 | 0.002 | 0.0004 | 0.0016 | 7368 |
| sample 6 | 0.006 | 0.0012 | 0.0048 | 8294 |
| sample 6 | 0.02 | 0.004 | 0.016 | 6506 |

### (Experimental Example 3: Continuous expansion culture of human adipose tissue-derived mesenchymal stem cells by 3D culture using mixture of chitin nanofiber and chitosan nanofiber)

α chitin nanofiber or chitosan nanofiber (biomass nanofiber BiNFi-S 2 mass %, Sugino Machine Limited) prepared according to WO 2015/111686 was suspended in ultrapure water (Milli-Q water) to 1% (w/v), dispersed by inversion mixing, and the aqueous solutions were autoclave sterilized at 121°C for 20 min. Used for the blending were α chitin nanofiber nanofiberized under condition of 200 MPa, pass 5 times (sample 1) and chitosan nanofibers nanofiberized under condition of 200 MPa, pass 5 times (sample 2). Furthermore, sample 1 and sample 2 were mixed such that the ratio (volume) of sample 1 and sample 2 was 50%:50% (sample 3), 25%:75% (sample 4), 20%:80% (sample 6), 15%:85% (sample 7) to give chitin nanofiber/chitosan nanofiber mixtures (samples 3, 4, 6, 7).

Medium compositions were prepared by adding samples 1 - 4, 6, 7 obtained above to a mesenchymal stem cell proliferation medium (C-28009, manufactured by Takara Bio Inc.), which is a serum medium, to a final concentration of 0.006%(w/v). The final concentrations of chitin nanofiber and/or chitosan nanofiber in the prepared medium compositions were as follows:
(1) Medium composition containing sample 1 (chitin nanofiber:0.006%(w/v), chitosan nanofiber:0%(w/v))
(2) Medium composition containing sample 2 (chitin nanofiber:0%(w/v), chitosan nanofiber:0.006%(w/v))
(3) Medium composition containing sample 3 (chitin nanofiber:0.003%(w/v), chitosan nanofiber:0.003%(w/v))
(4) Medium composition containing sample 4 (chitin nanofiber:0.0015%(w/v), chitosan nanofiber:0.0045%(w/v))
(5) Medium composition containing sample 6 (chitin nanofiber:0.0012%(w/v), chitosan nanofiber:0.0048%(w/v))
(6) Medium composition containing sample 7 (chitin nanofiber:0.0009%(w/v), chitosan nanofiber:0.0051%(w/v))

Samples include samples with chitin nanofiber/chitosan nanofiber ratios outside the scope of the present invention for comparative purposes.

Successively, the cultured human adipose-derived mesenchymal stem cells (C-12977, manufactured by Takara Bio Inc.) were suspended in each of the above-mentioned medium compositions at 13333 cells/mL, and seeded in a 96-well flat bottom ultra low attachment surface microplate (manufactured by Corning Incorporated, #3474) at 150 µL/well. The cells were cultured in a CO₂ incubator (37°C, 5% CO₂) in a static state for 5 days. An ATP reagent (150 µL, CellTiter-Glo^{™} Luminescent Cell Viability Assay, manufactured by Promega) was added to the culture medium on seeding (day 0) and day 5 after seeding and suspended and the mixture was stood for about 10 min at room temperature. The luminescence intensity (RLU value) was measured by FlexStation3 (manufactured by Molecular Devices) and the luminescence value of the medium alone was subtracted to calculate the number (mean of 3 samples) of viable cells.

On day 5 after seeding, nanofibers with cells adhered thereto were dispersed by pipetting, the suspension was recovered from each 96-well flat bottom ultra low attachment surface microplate, mixed with a mesenchymal stem cell proliferation medium (600 µL) containing each new sample (0.006%) by pipetting, and seeded in a 24-well flat bottom ultra low attachment surface microplate (manufactured by Corning Incorporated, #3473) at total amount/well (24 well microplate seeding day 0). The cell culture media on day 5 and day 10 after seeding in the 24 well microplate were each recovered in a 1.5 mL tube, and centrifuged at 2800 g for 3 min to cause sedimentation of cell-nanofiber part. To the cell-nanofiber pellets were added and suspended mesenchymal stem cell proliferation medium (150 µL) and ATP reagent (150 µL, CellTiter-Glo^{™} Luminescent Cell Viability Assay, manufactured by Promega), and the suspension was stood for about 10 min at room temperature. The luminescence intensity (RLU value) was measured by FlexStation3 (manufactured by Molecular Devices) and the luminescence value of the medium alone was subtracted to calculate the number (mean of 3 samples) of viable cells.

On day 10 after seeding in the 24-well microplate, nanofibers with cells adhered thereto were dispersed by pipetting, the suspension was each recovered from the 24-well flat bottom ultra low attachment surface microplate, mixed with a mesenchymal stem cell proliferation medium (3000 µL) containing each new sample (0.006%) by pipetting, and seeded in a 6-well flat bottom ultra low attachment surface microplate (manufactured by Corning Incorporated, #3471) at total amount/well (6 well microplate seeding day 0).

The cell culture medium on day 8 after seeding on the 6-well microplate was suspended by pipetting, and the total amount was adjusted to 3500 µL by adding the mesenchymal stem cell proliferation medium in consideration of the evaporation amount. 500 µL of the cell culture medium and ATP reagent (500 µL, CellTiter-Glo^{™} Luminescent Cell Viability Assay, manufactured by Promega) were added and suspended, and the suspension was stood for about 10 min at room temperature. The luminescence intensity (RLU value) was measured by FlexStation3 (manufactured by Molecular Devices) and the luminescence value of the medium alone was subtracted to calculate the number (mean of 3 samples) of viable cells. The final ATP value was converted to that of the total amount of 3500 µL.

As a result, when human adipose-derived mesenchymal stem cells were cultured using a medium composition containing α chitin nanofiber and chitosan nanofiber in which 15 wt% - 50 wt% of the total amount of α chitin nanofiber and chitosan nanofiber contained in the medium composition was α chitin nanofiber, it was possible to repeat loose aggregation of fibers and their dispersion by pipetting. In addition, by adding a medium composition containing fresh α-chitin nanofiber and chitosan nanofiber, expansion culture could be performed without using trypsin. Particularly, when 15 wt% - 20 wt% of the total amount of α chitin nanofiber and chitosan nanofiber contained in the medium composition was α chitin nanofiber, a decrease in the cell proliferation rate due to aggregation of human adipose-derived mesenchymal stem cells did not occur easily on nanofiber during continuous expansion culture, and a good proliferation promoting effect was shown even in a 6-well plate. The RLU values (ATP measurement, luminescence intensity) in respective cultures are shown in Table 5.

**[Table 5]**

| | day 0 (96 well) | day 5 (96 well) | day 5 (24 well) | day 10 (24 well) | day 8 (6 well) conversion |
|---|---|---|---|---|---|
| sample 3 | 1761 | 8188 | 16690 | 91054 | 126474 |
| sample 4 | 1823 | 8176 | 18165 | 73453 | 162159 |
| sample 6 | 1755 | 7159 | 16400 | 71173 | 201973 |
| sample 7 | 1823 | 6305 | 17364 | 65044 | 210427 |

### (Experimental Example 4: Stem cell marker expression variation of human adipose-derived mesenchymal stem cell by 3D culture using chitosan nanofiber or mixture of chitin nanofiber and chitosan nanofiber)

α chitin nanofiber or chitosan nanofiber (biomass nanofiber BiNFi-S 2 mass %, Sugino Machine Limited) prepared according to WO 2015/111686 was suspended in ultrapure water (Milli-Q water) to 1% (w/v), dispersed by inversion mixing, and the aqueous solution was autoclave sterilized at 121°C for 20 min. The α chitin nanofibers used were α chitin nanofiber nanofiberized under condition of 200 MPa, pass 5 times (sample 1). The chitosan nanofibers were chitosan nanofibers nanofiberized under condition of 200 MPa, pass 5 times (sample 2). Furthermore, sample 1 and sample 2 were mixed such that the ratio (volume) of sample 1 and sample 2 was 50%:50% (sample 3), 33%:67% (sample 4), 25%:75% (sample 5) to give chitin nanofiber/chitosan nanofiber mixtures (samples 3-5). Medium compositions were prepared by adding samples 1 - 5 obtained above to a mesenchymal stem cell proliferation medium 2 medium (C-28009, manufactured by Takara Bio Inc.) to a final concentration of 0.015%(w/v), and no addition medium composition (sample 11) which was free of the above-mentioned base material was prepared. The final concentrations of chitin nanofiber and/or chitosan nanofiber in the prepared medium compositions were as follows:
(1) Medium composition containing sample 1 (chitin nanofiber:0.015%(w/v), chitosan nanofiber:0%(w/v))
(2) Medium composition containing sample 2 (chitin nanofiber:0%(w/v), chitosan nanofiber:0.015%(w/v))
(3) Medium composition containing sample 3 (chitin nanofiber:0.0075%(w/v), chitosan nanofiber:0.0075%(w/v))
(4) Medium composition containing sample 4 (chitin nanofiber:0.005%(w/v), chitosan nanofiber:0.01%(w/v))
(5) Medium composition containing sample 5 (chitin nanofiber:0.00375%(w/v), chitosan nanofiber:0.01125%(w/v))
Samples include samples with chitin nanofiber/chitosan nanofiber ratios outside the scope of the present invention for comparative purposes.

Successively, the cultured human adipose-derived mesenchymal stem cells (C-12977, manufactured by Takara Bio Inc.) were seeded in the above-mentioned medium composition added with chitin and/or chitosan nanofiber or no addition medium composition at 50000 cells/mL, and dispensed in the wells of a 6-well flat bottom ultra low attachment surface microplate (#3471, manufactured by Corning Incorporated) at 2 mL per well. As a comparison, 25000 cells/mL were seeded in the no addition medium composition, and dispensed in the wells of a 6-well flat bottom attachment surface microplate (#3516, manufactured by Corning Incorporated) at 2 mL per well (sample 12). Each plate was cultured in a standing still state in a CO₂ incubator (37°C, 5%CO₂) continuously for 6 days. On day 6, the cells were recovered, and RLT solution (350 µL, RNeasy mini kit (manufactured by QIAGEN, #74106)) was added to give an RNA extraction solution. To the RNA extraction solution was added 70% ethanol (350 µL), and the mixture was added to RNeasy spin column and centrifuged at 8000xg for 15 sec. Successively, 700 µL of RW1 solution was added to RNeasy spin column, and centrifuged at 8000xg for 15 sec. Successively, 500 µL of RPE solution was added, and centrifuged at 8000xg for 15 sec. Furthermore, 500 µL of RPE solution was added, and centrifuged at 8000xg for 2 min. RNase-free solution was added to RNAs remaining in the RNeasy spin column to elute them. Then, cDNAs were synthesized from the obtained RNAs by using PrimeScript RT reagent Kit (Perfect Real Time) (manufactured by Takara Bio Inc., #RR037A). Using the synthesized cDNAs, Premix EX Taq (Perfect Real Time) (manufactured by Takara Bio Inc., #RR039A), and Taq man Probe (manufactured by Applied Bio Systems), real-time PCR was performed. As the Taq man Probes (manufactured by Applied Bio Systems), OCT4 used was Hs04260367_gH, SOX2 used was Hs01053049_s1, NANOG used was Hs04399610_g1, CXCR4 used was Hs00607978 s1, and GAPDH used was Hs99999905_m1. As the instrument, real-time PCR7500 was used. In the analysis, relative values obtained by amending the values of each target gene with the values of GAPDH were calculated and compared.

As a result, when human adipose-derived mesenchymal stem cells were cultured in a medium composition containing a mixture of chitosan nanofiber and α chitin nanofiber and chitosan nanofiber in a 6-well flat bottom ultra low attachment surface microplate, an increase in the relative expression levels of OCT4, SOX2, and NANOG genes showing an undifferentiated state and CXCR4 gene showing homing-related chemotacticity was observed. On the other hand, a clear increase in the expression level of these genes was not observed under the conditions of monolayer culture on the adhesive plate and 3D culture using only chitin nanofibers on the low adhesive plate. The relative values of each gene expression are shown in Table 6.

**[Table 6]**

| | | SOX2 | OCT4 | NANOG | CXCR4 |
|---|---|---|---|---|---|
| low adhesive plate | no addition (sample 11) | 0.04 | 0.05 | 0.05 | 0.4 |
| | sample 1 | 0.04 | 0.05 | 0.05 | 0.4 |
| | sample 2 | 1.04 | 0.79 | 0.87 | 10.7 |
| | sample 3 | 0.21 | 0.22 | 0.2 | 2.4 |
| | sample 4 | 0.12 | 0.23 | 0.19 | 2.6 |
| | sample 5 | 0.22 | 0.36 | 0.26 | 5.7 |
| adhesive plate | no addition (sample 12) | 0.03 | 0.05 | 0.05 | 0.7 |

The property values (measured concentration, viscosity, median particle size and average particle size and the like of α chitin or chitosan) of the α chitin nanofiber dispersion and chitosan nanofiber dispersion prepared under various fibrilating conditions (pressure, grinding number) used in Experimental Examples 1 - 4 (and below-mentioned Experimental Examples 5, 6, 7) are shown in Table 7.

**[Table 7]**

| nanofiber starting material | pressure (MPa) | fibrillating number (Pass) | measured concentration % (w/v) | viscosity mPa·S (0-20°C) | laser diffraction average particle size (µm) | laser diffraction median particle size (µm) |
|---|---|---|---|---|---|---|
| chitin | 200 | 5 | 1.05% | 6.73 | 80.7 | 65 |
| chitosan | 200 | 5 | 0.94% | 3.95 | 51.2 | 44.3 |

### (Experimental Example 5: Continuous expansion culture of human umbilical cord-derived mesenchymal stem cells by 3D culture using mixture of chitin nanofiber and chitosan nanofiber)

Medium compositions were prepared by adding sample 6 obtained above to a mesenchymal stem cell proliferation medium (C-28009, manufactured by Takara Bio Inc.), which is a serum medium, to a final concentration of 0.05%(w/v) (the medium composition prepared here is hereinafter sometimes to be referred to as "nanofiber-containing mesenchymal stem cell proliferation medium"). The final concentrations of chitin nanofiber and chitosan nanofiber in the prepared nanofiber-containing mesenchymal stem cell proliferation medium were as follows:
chitin nanofiber:0.01%(w/v), chitosan nanofiber:0.04%(w/v).

Successively, the cultured human umbilical cord-derived mesenchymal stem cells (C-12971, manufactured by Takara Bio Inc.) were suspended in the above-mentioned nanofiber-containing mesenchymal stem cell proliferation medium at 15000 cells/mL, and seeded in a 6-well flat bottom ultra low attachment surface microplate (manufactured by Corning Incorporated, #3471) at 10 mL/well. The cells were cultured in a CO₂ incubator (37°C, 5% CO₂) in a static state for 3 days. During culture, the cells adhered to the nanofibers precipitated on the bottom of the culture well; however, they did not adhere to the surface of the culture well. On day 3, about 5 mL of the medium supernatant in the well substantially free of nanofiber/cell precipitates was removed, a fresh nanofiber-containing mesenchymal stem cell proliferation medium (5 mL) was added, suspended by pipetting, and culture was continued until day 7. An ATP reagent (300 µL, CellTiter-Glo^{™} Luminescent Cell Viability Assay, manufactured by Promega) was added and suspended in the culture medium (300 µL) on seeding (day 0) and day 7 after seeding, and the suspension was stood for about 10 min at room temperature. The luminescence intensity (RLU value) was measured by FlexStation3 (manufactured by Molecular Devices) and the luminescence value of the medium alone was subtracted to calculate the number (mean of 2 samples) of viable cells.

On day 7 after seeding, nanofibers with cells adhered thereto were dispersed by pipetting, the suspension was recovered from 6-well flat bottom ultra low attachment surface microplate, mixed with a fresh nanofiber-containing mesenchymal stem cell proliferation medium (40 mL) by pipetting, and seeded in a 125 mL flask (manufactured by Thermo Scientic, 4115-0125) at total amount/flask and cultured. On day 10, about 25 mL of the medium in the flask was removed, a fresh nanofiber-containing mesenchymal stem cell proliferation medium (25 mL) was added, suspended by pipetting, and culture was continued until day 14 after seeding. An ATP reagent (500 µL, CellTiter-Glo^{™} Luminescent Cell Viability Assay, manufactured by Promega) was added and suspended in the cell culture medium (500 µL) on day 7 after expansion of the 125 mL flask, and the suspension was stood for about 10 min at room temperature. The luminescence intensity (RLU value) was measured by FlexStation3 (manufactured by Molecular Devices) and the luminescence value of the medium alone was subtracted to calculate the number (mean of 2 samples) of viable cells. The final ATP value in the flask was converted using the expansion ratio from 6 wells to the flask (5-fold).

As a result, when human umbilical cord-derived mesenchymal stem cells were cultured using a medium composition containing α chitin nanofiber and chitosan nanofiber, it was possible to repeat loose aggregation of fibers and their dispersion by pipetting. In addition, by adding a medium composition containing fresh α-chitin nanofiber and chitosan nanofiber, expansion culture could be performed without using trypsin. The RLU values (ATP measurement, luminescence intensity) in respective cultures are shown in Table 8.

**[Table 8]**

| | day 0 (6 well) | day 7 (6 well) | day 14 (125 mL flask) |
|---|---|---|---|
| sample 6 | 1935 | 21036 | 73764 |

### (Experimental Example 6: Suspension culture of human umbilical cord-derived mesenchymal stem cell at low serum concentration in medium composition containing chitin nanofiber and chitosan nanofiber, and continuous recovery of cell secretion products - 1)

In Experimental Example 5, 125 mL of the flask culture medium on day 14 of culture of human umbilical cord-derived mesenchymal stem cells using a medium composition containing α chitin nanofiber and chitosan nanofiber (i.e., nanofiber-containing mesenchymal stem cell proliferation medium) was used. On day 14, about 25 mL of the medium supernatant substantially free of nanofiber/cell precipitates in the flask was removed, 25 mL of a fresh DMEM medium (044-29765, FUJIFILM Wako Pure Chemical Corporation, free of nanofiber or serum) was added, suspended by pipetting, and culture was continued until day 17. On day 17, about 25 mL of the medium supernatant substantially free of nanofiber/cell precipitates in the flask was further removed, 25 mL of a fresh DMEM medium (044-29765, FUJIFILM Wako Pure Chemical Corporation, free of nanofiber and serum) was added to the well, suspended by pipetting, and culture was continued until day 21. Thereafter, about 25 mL of the medium supernatant in the flask was removed on days 24, 28, 31, 35, 25 mL of a fresh DMEM medium: mesenchymal stem cell proliferation medium (3:1) (all media free of nanofiber) was added, suspended by pipetting, and culture was continued until day 35.

On days 21, 24, 28, 31, and 35, the recovered medium supernatant was centrifuged (400 g, 3 min), and then the culture supernatant was recovered. The recovered culture supernatant was stored at -80°C until use. The pellets (nanofiber/cell, etc.) after centrifugation were returned to the flask used under cell culture, and the culture in the flask was continued. That is, the concentration of nanofiber in the medium is always constant throughout the above-mentioned all steps.

On days 14, 21, 28, and 35, 500 µL of ATP reagent (CellTiter-Glo^{™} Luminescent Cell Viability Assay, manufactured by Promega) was added to 500 µL of cell culture medium in a 125 mL flask, suspended, and allowed to stand at room temperature for about 10 min. The luminescence intensity (RLU value) was measured by FlexStation3 (manufactured by Molecular Devices) and the luminescence value of the medium alone was subtracted to calculate the number of viable cells (mean of 2 samples).

### [Measurement of amount of exosome produced by enzyme antibody method]

The amount of exosome produced in each culture supernatant (days 17, 21, 24, 28, 31, 35) was measured by an enzyme antibody method (ELISA; enzyme-linked immunosorbent assay). For the measurement, PS Capture^{™} Exosome ELISA Kit (manufactured by Wako Pure Chemical Industries, Ltd., #297-79201) was used. An operation of adding a reaction/washing solution at 300 µL/well to Exosome Capture 96 Well Plate was repeated 3 times. A 10-fold diluted culture supernatant was dispensed at 100 µL/well and reacted using a microplate shaker at room temperature for 2 hr. After completion of the reaction, the reaction mixture was discarded, and an operation of adding a reaction/washing solution at 300 µL/well to each well was repeated 3 times. A control anti-CD63 antibody reaction solution for detection was dispensed at 100 µL/well, and reacted using a microplate shaker at room temperature for 1 hr. After completion of the reaction, the reaction mixture was discarded, and an operation of adding a reaction/washing solution at 300 µL/well to each well was repeated 3 times. A secondary antibody reaction solution for detection was dispensed at 100 µL/well, and reacted using a microplate shaker at room temperature for 1 hr. After completion of the reaction, the reaction mixture was discarded, and an operation of adding a reaction/washing solution at 300 µL/well to each well was repeated 5 times. TMB Solution was dispensed at 100 µL/well, and reacted at room temperature for 30 min. After completion of the reaction, Stop Solution was added at 100 µL/well, and absorbance at 450 nm and 620 nm was measured. The absorbance value of each sample was the value obtained by subtracting the absorbance at 620 nm from the absorbance at 450 nm (ΔAbs).

As a result, the viable cell number of human umbilical cord-derived mesenchymal stem cells proliferated on the medium composition containing α chitin nanofiber and chitosan nanofiber was maintained up to day 35 even in a low serum medium with a ratio of the mesenchymal stem cell medium of 25%. The production amount of the CD63 value (exosome) in each culture supernatant was higher when the ratio of the mesenchymal stem cell medium was 25% than that of 50%. The production amount increased as the culture was continued in the low serum medium of 25%. The RLU values (ATP measurement, luminescence intensity) in each culture are shown in Table 9, and the ΔAbs value by CD63 is shown in Table 10.

**[Table 9]**

| | day 14 | day 21 | day 28 | day 35 |
|---|---|---|---|---|
| sample 6 | 14753 | 14934 | 16439 | 15320 |

**[Table 10]**

| | recovered on day 14 - 17 | recovered on day 17 - 21 | recovered on day 21 - 24 |
|---|---|---|---|
| mesenchymal stem cell proliferation medium rate (%) | 50 | 25 | 25 |
| serum concentration (%) | 1 | 0.5 | 0.5 |
| sample 6 | 1.19 | 1.24 | 1.77 |

| | recovered on day 24 - 28 | recovered on day 28 - 31 | recovered on day 31 - 35 |
|---|---|---|---|
| mesenchymal stem cell proliferation medium rate (%) | 25 | 25 | 25 |
| serum concentration (%) | 0.5 | 0.5 | 0.5 |
| sample 6 | 1.99 | 1.87 | 2.5 |

| medium | buffer |
|---|---|
| 0.12 | 0.12 |

### (Experimental Example 7: Suspension culture of human umbilical cord-derived mesenchymal stem cell in step-by step reduction of serum in medium composition containing chitin nanofiber and chitosan nanofiber, and recovery of cell secretion products - 2)

Medium compositions were prepared by adding samples 3 and 6 obtained above to a mesenchymal stem cell proliferation medium (C-28009, manufactured by Takara Bio Inc.), which is a serum medium, to a final concentration of 0.05%(w/v) (the medium composition prepared here is hereinafter sometimes to be referred to as "nanofiber-containing mesenchymal stem cell proliferation medium"). The final concentrations of chitin nanofiber and chitosan nanofiber in the prepared nanofibercontaining mesenchymal stem cell proliferation medium were as follows:
(1) Medium composition containing sample 3 (chitin nanofiber:0.025%(w/v), chitosan nanofiber:0.025%(w/v))
(2) Medium composition containing sample 6 (chitin nanofiber:0.01%(w/v), chitosan nanofiber:0.04%(w/v))

Successively, the cultured human umbilical cord-derived mesenchymal stem cells (C-12971, manufactured by Takara Bio Inc.) were suspended in the above-mentioned nanofiber-containing mesenchymal stem cell proliferation medium at 15000 cells/mL, and seeded in a 24-well flat bottom ultra low attachment surface microplate (manufactured by Corning Incorporated, #3473) at 1.2 mL/well. The cells were cultured in a CO₂ incubator (37°C, 5% CO₂) in a static state for 3 days. During culture, the cells adhered to the nanofibers precipitated on the bottom of the culture well; however, they did not adhere to the surface of the culture well. On day 3, about 0.6 mL of the medium supernatant in the well substantially free of nanofiber/cell precipitates was removed, 0.6 mL of a mesenchymal stem cell proliferation medium was added, suspended by pipetting, and culture was continued until day 6. On day 6 after seeding, nanofibers with cells adhered thereto were dispersed by pipetting, the suspension was recovered from 24-well flat bottom ultra low attachment surface microplate, mixed with a fresh nanofiber-containing mesenchymal stem cell proliferation medium (8.4 mL) by pipetting, and seeded in a 6-well flat bottom ultra low attachment surface microplate (manufactured by Corning Incorporated, #3473) at 9.6 mL/well. The cells were cultured in a CO₂ incubator (37°C, 5% CO₂) in a static state for 4 days. On day 10, about 5 mL of the medium supernatant in the well was removed, 5 mL of a mesenchymal stem cell proliferation medium (free of nanofiber) was added, suspended by pipetting, and culture was continued until day 14. On day 14 after seeding, nanofibers with cells adhered thereto were dispersed by pipetting, the suspension was recovered from 6-well flat bottom ultra low attachment surface microplate, mixed with a fresh nanofiber-containing mesenchymal stem cell proliferation medium (about 40 mL) by pipetting, and seeded in a 125 mL flask (manufactured by Thermo Scientic, 4115-0125) at total amount/flask and cultured. On day 17, about 25 mL of the medium supernatant in the flask was removed, a fresh mesenchymal stem cell proliferation medium (25 mL, free of nanofiber) was added, suspended by pipetting, and culture was continued until day 20 after seeding.

On day 20, about 25 mL of the medium supernatant in the flask was removed, 25 mL of a fresh DMEM medium (044-29765, FUJIFILM Wako Pure Chemical Corporation, free of nanofiber and serum) was added, suspended by pipetting, and culture was continued until day 23. On day 23, about 25 mL of the medium supernatant in the flask was further removed, 25 mL of a fresh DMEM medium (044-29765, FUJIFILM Wako Pure Chemical Corporation, free of nanofiber or serum) was added to the well, suspended by pipetting, and culture was continued until day 26. Thereafter, about 25 mL of the medium supernatant in the flask was removed on days 29, 32, and 35, 25 mL of a fresh DMEM medium (free of nanofiber and serum) was added, suspended by pipetting, and culture was continued until day 35.

On days 26, 29, 32, and 35, the recovered medium was centrifuged (400 g, 3 min), and then the culture supernatant was recovered. The recovered culture supernatant was stored at -80°C until use. The pellets (nanofiber/cell, etc.) after centrifugation were returned to the flask used under cell culture, and the culture in the flask was continued. That is, the concentrations of nanofiber in the medium were always constant throughout the above-mentioned all steps.

On days 26, 29, 32, and 35, 500 µL of ATP reagent (CellTiter-Glo^{™} Luminescent Cell Viability Assay, manufactured by Promega) was added to 500 µL of cell culture medium in a 125 mL flask, suspended, and allowed to stand at room temperature for about 10 min. The luminescence intensity (RLU value) was measured by FlexStation3 (manufactured by Molecular Devices) and the luminescence value of the medium alone was subtracted to calculate the number of viable cells (mean of 2 samples).

### [Measurement of amount of exosome produced by enzyme antibody method]

The amount of exosome produced in each culture supernatant (days 26, 29, 32, 35) was measured by an enzyme antibody method (ELISA; enzyme-linked immunosorbent assay). For the measurement, PS Capture^{™} Exosome ELISA Kit (manufactured by Wako Pure Chemical Industries, Ltd., #297-79201) was used. An operation of adding a reaction/washing solution at 300 µL/well to Exosome Capture 96 Well Plate was repeated 3 times. A 10-fold diluted culture supernatant was dispensed at 100 µL/well and reacted using a microplate shaker at room temperature for 2 hr. After completion of the reaction, the reaction mixture was discarded, and an operation of adding a reaction/washing solution at 300 µL/well to each well was repeated 3 times. A control anti-CD63 antibody reaction solution for detection was dispensed at 100 µL/well, and reacted using a microplate shaker at room temperature for 1 hr. After completion of the reaction, the reaction mixture was discarded, and an operation of adding a reaction/washing solution at 300 µL/well to each well was repeated 3 times. A secondary antibody reaction solution for detection was dispensed at 100 µL/well, and reacted using a microplate shaker at room temperature for 1 hr. After completion of the reaction, the reaction mixture was discarded, and an operation of adding a reaction/washing solution at 300 µL/well to each well was repeated 5 times. TMB Solution was dispense at 100 µL/well, and reacted at room temperature for 30 min. After completion of the reaction, Stop Solution was added at 100 µL/well, and absorbance at 450 nm and 620 nm was measured. The absorbance value of each sample was the value obtained by subtracting the absorbance at 620 nm from the absorbance at 450 nm (ΔAbs).

As a result, as the medium gradually became a low serum medium by the addition of the DMEM medium to the human umbilical cord-derived mesenchymal stem cells that proliferated on the medium composition containing α chitin nanofiber and chitosan nanofiber, the number of viable cells decreased when the proportion of the mesenchymal stem cell medium containing serum reached 6.25%. On the other hand, as for the CD63 value (exosome) in each culture supernatant, the production amount decreased as the serum level became lower and the production amount was the highest under the condition where the proportion of the mesenchymal stem cell medium was 25% (that is, the serum concentration was 0.5% wt%). The RLU values (ATP measurement, luminescence intensity) in each culture are shown in Table 11, and the ΔAbs values by CD63 are shown in Table 12.

**[Table 11]**

| | day 26 | day 29 | day 32 | day 35 |
|---|---|---|---|---|
| sample 3 | 894900 | 886450 | 568208 | 441348 |
| sample 6 | 527673 | 750692 | 410465 | 437438 |

**[Table 12]**

| | recovered on day 23 - 26 | recovered on day 26 - 29 | recovered on day 29 - 32 |
|---|---|---|---|
| mesenchymal stem cell proliferation medium rate (%) | 25 | 12.5 | 6.25 |
| serum concentration (%) | 0.5 | 0.25 | 0.125 |
| sample 3 | 1.64 | 1.11 | 0.99 |
| sample 6 | 1.03 | 1.01 | 0.93 |

| | recovered on day 32 - 35 |
|---|---|
| mesenchymal stem cell proliferation medium rate (%) | 3.13 |
| serum concentration (%) | 0.063 |
| sample 3 | 0.36 |
| sample 6 | 0.51 |

### (Experimental Example 8: Amount of PGE2 produced by 3D culture of human bone marrow-derived and human umbilical cord-derived mesenchymal stem cells by using mixture of chitin nanofiber and chitosan nanofiber)

A medium composition was prepared by adding chitin nanofiber and chitosan nanofiber to mesenchymal stem cell proliferation medium 2 medium (C-28009, manufactured by Takara Bio Inc.) (final concentration: 0.05%(w/v)) (sample 6), and a no addition medium composition (sample 12) which did not contain the above-mentioned base material was prepared. Successively, the cultured human bone marrow-derived mesenchymal stem cells (C-12974, manufactured by Takara Bio Inc.) were seeded at 40000 cells/mL, and human umbilical cord-derived mesenchymal stem cells (C-12971, manufactured by Takara Bio Inc.) were seeded at 80000 cells/mL in the above-mentioned medium composition added with chitin and chitosan nanofiber or no addition medium composition, and the medium composition added with chitin and chitosan nanofiber was dispensed in a 24-well flat bottom ultra low attachment surface microplate (#3473, manufactured by Corning Incorporated) at 1 mL per well. As a comparison, the cells were seeded in the wells of 24-well flat bottom attachment surface microplate (#3526, manufactured by Corning Incorporated) or 6-well flat bottom attachment surface microplate (#3516, manufactured by Corning Incorporated) at 1 mL or 2 mL per well. Each plate was cultured in a standing still state in a CO₂ incubator (37°C, 5%CO₂) continuously for 2 days. On day 2, the medium composition added with chitin and chitosan nanofiber and no addition medium composition were transferred from each well to a 15 mL tube, centrifuged at 300xg for 3 min, and the culture supernatant was recovered. The culture supernatant was removed, 1 mL of mesenchymal stem cell proliferation medium 2 medium or mesenchymal stem cell proliferation medium 2 medium containing TNF-α (#210-TA, manufactured by R&D systems) at a final concentration of 10 ng/mL was added to the 15 mL tube, and the medium composition added with chitin and chitosan nanofiber was placed back in the wells of 24-well flat bottom ultra low attachment surface microplate (#3473, manufactured by Corning Incorporated). The medium was removed from the 24-well flat bottom attachment surface microplate (#3526, manufactured by Corning Incorporated) and 1 mL of mesenchymal stem cell proliferation medium 2 medium or mesenchymal stem cell proliferation medium 2 medium containing TNF-α at a final concentration of 10 ng/mL was added. Each plate was cultured in a CO₂ incubator (37°C, 5% CO₂) in a static state for 1 day. On day 1, the medium composition added with chitin and chitosan nanofiber and no addition medium composition were transferred from each well to a 15 mL tube, centrifuged at 300xg for 3 min, and the culture supernatant was recovered. To evaluate the cell number here, an ATP reagent (1 mL, CellTiter-Glo (registered trade mark) Luminescent Cell Viability Assay, manufactured by Promega) was added and suspended in each sample after recovery of the culture supernatant, and the suspension was stood for about 10 min at room temperature. The luminescence intensity (RLU value) was measured by Enspire (manufactured by Perkin Elmer) and the number of viable cells was measured by subtracting the luminescence value of the medium alone. Then, PGE2 contained in the recovered culture supernatant was quantified using PGE2 ELISA kit (#ADI-900-001, manufactured by EnzoLife Science). The standard diluted with Assay Buffer and the culture supernatant (100 µL) were added to each well of the 96-well plate included in the kit. Successively, 50 µL of blue conjugate was added to each well. Furthermore, 50 µL of yellow antibody was added to each well, and the mixture was shaken under room temperature conditions for 2 hr. Successively, the solution was discarded, a wash solution was added at 400 µL/well, and the solution was discarded. The above-mentioned operation was repeated 3 times. 200 µL of pNpp substrate solution was added to each well, and the mixture was shaken under room temperature conditions for 45 min. Finally, 50 µL of stop solution was added to quench the reaction, and the absorbance at 405 nm was measured. The concentration of PGE2 contained in each sample was calculated from 4-parameter logistic regression of the calibration curve. To calculate the amount of secretion per number of cells, a relative value was calculated by dividing the calculated amount of PGE2 by the luminescence intensity.

As a result, the relative value of the amount of PGE2 secretion increased more in the 3D culture in which the medium composition added with chitin nanofiber and chitosan nanofiber and the low adhesive plate were combined than in the monolayer culture using the no addition medium composition and the adhesive plate. Also, in the comparison of TNF-α treatment, the 3D culture in which the medium composition added with chitin nanofiber and chitosan nanofiber and the low adhesive plate were combined was higher than the monolayer culture using the no addition medium composition and the adhesive plate. The relative values of human bone marrow-derived and human umbilical cord-derived mesenchymal stem cells in each sample are shown in Table 13 and Table 14.

**[Table 13]**

| human bone marrow-derived mesenchymal stem cell | | | relative value (PGE2/Celltiter |
|---|---|---|---|
| adhesive plate | no addition (sample 12) | control | 1.59E-08 |
| | | TNF-α 10 ng/mL | 1.19E-07 |
| low adhesive plate | sample 6 | control | 5.87E-06 |
| | | TNF-α 10 ng/mL | 1.12E-05 |

**[Table 14]**

| human umbilical cord-derived mesenchymal stem cell | | | relative value (PGE2/Celltiter |
|---|---|---|---|
| adhesive plate | no addition (sample 12) | control | 4.19E-06 |
| | | TNF-α 20 ng/mL | 1.91E-05 |
| low adhesive plate | sample 6 | control | 0.00252 |
| | | TNF-α 20 ng/mL | 0.00424 |

### (Experimental Example 9: Amount of bFGF produced by 3D culture of human bone marrow-derived and human adipose-derived mesenchymal stem cells by using mixture of chitin nanofiber and chitosan nanofiber)

A medium composition was prepared by adding chitin nanofiber and chitosan nanofiber to mesenchymal stem cell proliferation medium 2 medium (C-28009, manufactured by Takara Bio Inc.) (final concentration: 0.05%(w/v)) (sample 6), and a no addition medium composition (sample 12) which dose not contain the above-mentioned base material was prepared. Successively, the cultured human bone marrow-derived mesenchymal stem cells (C-12974, manufactured by Takara Bio Inc.) were seeded at 80000 cells/mL, and human adipose-derived mesenchymal stem cells (C-12977, manufactured by Takara Bio Inc.) were seeded at 100000 cells/mL in the above-mentioned medium composition added with chitin and chitosan nanofiber or no addition medium composition, and the medium composition added with chitin and chitosan nanofiber was dispensed in a 24-well flat bottom ultra low attachment surface microplate (#3473, manufactured by Corning Incorporated) at 1 mL per well. As a comparison, the cells were seeded in the wells of 24-well flat bottom attachment surface microplate (#3526, manufactured by Corning Incorporated) at 1 mL per well. Each plate was cultured in a standing still state in a CO₂ incubator (37°C, 5%CO₂) continuously for 2 days. On day 2, the medium composition added with chitin and chitosan nanofiber and no addition medium composition were transferred from each well to a 15 mL tube, centrifuged at 300xg for 3 min, and the culture supernatant was removed. Successively, 1 mL of 17% FBS-containing MEMα medium was added, and the medium composition added with chitin and chitosan nanofiber was placed back in the wells of 24-well flat bottom ultra low attachment surface microplate (#3473, manufactured by Corning Incorporated). In the no addition medium composition, after removal of the mesenchymal stem cell proliferation medium 2 medium, 1 mL of the 17% FBS-containing MEMα medium was added to the 24-well flat bottom attachment surface microplate (#3526, manufactured by Corning Incorporated). Each plate was cultured in a CO₂ incubator (37°C, 5% CO₂) in a static state for 1 day. On day 1, the medium composition added with chitin and chitosan nanofiber and no addition medium composition were transferred from each well to a 15 mL tube, centrifuged at 300xg for 3 min, and the culture supernatant was recovered. To evaluate the cell number here, an ATP reagent (1 mL, CellTiter-Glo (registered trade mark) Luminescent Cell Viability Assay, manufactured by Promega) was added and suspended in each sample after recovery of the culture supernatant, and the suspension was stood for about 10 min at room temperature. The luminescence intensity (RLU value) was measured by Enspire (manufactured by Perkin Elmer) and the number of viable cells was measured by subtracting the luminescence value of the medium alone. Successively, bFGF contained in the recovered culture supernatant was quantified using a bFGF ELISA kit (#ELH-bFGF-1, manufactured by RayBiotech). A standard or sample solution (100 µL) was added to a well and shaken for 2.5 hr at room temperature. Successively, the solution was discarded, and 300 µL of 1x wash solution was added and removed. The above-mentioned operation was repeated 4 times. 100 µL of 1x Detection antibody was added, and the mixture was shaken for 1 hr at room temperature. The solution was discarded, and 300 µL of 1x wash solution was added and removed. The above-mentioned operation was repeated 4 times. 100 µL of HRP-Streptavidin solution was added, and the mixture was shaken for 45 min at room temperature. Successively, the solution was discarded, and 300 µL of 1x wash solution was added and removed. The above-mentioned operation was repeated 4 times. 100 µL of TMB One-Step Substrate reagent was added, and the mixture was shaken in a dark place for 30 min at room temperature. Finally, 50 µL of stop solution was added, and the absorbance at 450 nm was measured. The concentration of bFGF contained in each sample was calculated from 4-parameter logistic regression of the calibration curve. To calculate the amount of secretion per number of cells, a relative value was calculated by dividing the calculated amount of bFGF by the luminescence intensity.

As a result, in both the human bone marrow-derived mesenchymal stem cells and human adipose-derived mesenchymal stem cells, the relative value of the amount of bFGF secretion increased more in the 3D culture in which the medium composition added with chitin nanofiber and chitosan nanofiber and the low adhesive plate were combined than in the monolayer culture using the no addition medium composition and the adhesive plate. The relative values of the human bone marrow-derived mesenchymal stem cells and human adipose-derived mesenchymal stem cells are shown in Table 15 and Table 16.

**[Table 15]**

| human bone marrow-derived mesenchymal stem cell | | relative value (bFGF/Celltiter glo) |
|---|---|---|
| adhesive plate | no addition (sample 12) | 0.00013 |
| low adhesive plate | sample 6 | 0.00381 |

**[Table 16]**

| human adipose-derived mesenchymal stem cell | | relative value (bFGF/Celltiter glo) |
|---|---|---|
| adhesive plate | no addition (sample 12) | 8.99E-05 |
| low adhesive plate | sample 6 | 0.00077 |

### (Experimental Example 10: Amount of TSG-6 produced by 3D culture of human bone marrow-derived and human umbilical cord-derived mesenchymal stem cells by using mixture of chitin nanofiber and chitosan nanofiber)

A medium composition was prepared by adding chitin nanofiber and chitosan nanofiber to mesenchymal stem cell proliferation medium 2 medium (C-28009, manufactured by Takara Bio Inc.) (final concentration: 0.05%(w/v)) (sample 6), and a no addition medium composition (sample 12) which does not contain the above-mentioned base material was prepared. Successively, the cultured human bone marrow-derived mesenchymal stem cells (C-12974, manufactured by Takara Bio Inc.) were seeded at 80000 cells/mL, and human umbilical cord-derived mesenchymal stem cells (C-12971, manufactured by Takara Bio Inc.) were seeded at 40000 cells/mL in the above-mentioned medium composition added with chitin and chitosan nanofiber or no addition medium composition, and the medium composition added with chitin and chitosan nanofiber was dispensed in a 24-well flat bottom ultra low attachment surface microplate (#3473, manufactured by Corning Incorporated) at 1 mL per well. As a comparison, the cells were seeded in the wells of 6-well flat bottom attachment surface microplate (#3516, manufactured by Corning Incorporated) or 24-well flat bottom attachment surface microplate (#3526, manufactured by Corning Incorporated) at 1 mL per well. Each plate was cultured in a standing still state in a CO₂ incubator (37°C, 5%CO₂) continuously for 2 days. On day 2, the medium composition added with chitin and chitosan nanofiber and no addition medium composition were transferred from each well to a 15 mL tube, centrifuged at 300xg for 3 min, and the culture supernatant was removed. After removal of the culture supernatant, 1 mL of a mesenchymal stem cell proliferation medium 2 medium or a mesenchymal stem cell proliferation medium 2 medium containing TNF-α (#210-TA, manufactured by R&D systems) at a final concentration of 10 ng/mL or 20 ng/mL was added to a 15 mL tube, and the medium composition added with chitin and chitosan nanofiber was placed back in the wells of 24-well flat bottom ultra low attachment surface microplate (#3473, manufactured by Corning Incorporated). To the 24-well flat bottom attachment surface microplate (#3526, manufactured by Corning Incorporated), after removal of the medium, 1 mL of a mesenchymal stem cell proliferation medium 2 medium or a mesenchymal stem cell proliferation medium 2 medium containing TNF-α at a final concentration of 10 ng/mL or 20 ng/mL was added. Each plate was cultured in a CO₂ incubator (37°C, 5% CO₂) in a static state for 1 day. On day 1, the medium composition added with chitin and chitosan nanofiber and no addition medium composition were transferred from each well to a 15 mL tube, centrifuged at 300xg for 3 min, and the culture supernatant was recovered. To evaluate the cell number here, an ATP reagent (1 mL, CellTiter-Glo (registered trade mark) Luminescent Cell Viability Assay, manufactured by Promega) was added and suspended in each sample after recovery of the culture supernatant, and the suspension was stood for about 10 min at room temperature. The luminescence intensity (RLU value) was measured by Enspire (manufactured by Perkin Elmer) and the number of viable cells was measured by subtracting the luminescence value of the medium alone. Successively, TSG-6 contained in the recovered culture supernatant was quantified using ELISA. TSG-6 antibody (#sc-65886, manufactured by Santacruz) diluted with 0.2M carbonic acid - bicarbonic acid buffer (pH9.2) to 10 ug/mL was added at 50 µL/well to Maxisorp flat bottom (#44-2404-21, manufactured by Thermo Fisher Scientific), and left standing at 4°C for 24 hr. After 24 hr, 300 µL of a PBST solution obtained by adding Tween-20 (#P7949, manufactured by Sigma Ltd. Aldrich) to D-PBS(-) (#043-29791, manufactured by FUJIFILM Wako Pure Chemical Corporation) to a final concentration of 0.05%(v/v) was added and removed. This operation was repeated 3 times. A PBST solution (100 uL) containing 5% BSA (#A2153, manufactured by Sigma Ltd. Aldrich) was added and the mixture was left standing at room temperature for 30 min. The solution was discarded, and 300 µL of the PBST solution was added and removed. This operation was repeated 3 times. Successively, 50 µL of TSG-6 (#2104-TS, manufactured by R&D Systems) prepared for calibration curve or samples to be evaluated was added to each well, and the mixture was left standing at room temperature for 2 hr. The solution was discarded, and 300 µL of the PBST solution was added and removed. This operation was repeated 3 times. 50 µL of a solution of Biotinylated anti human TSG-6 antibody (#BAF2104, manufactured by R&D Systems) diluted with PBST to 5 ug/mL was added, and the mixture was left standing at room temperature for 120 min. The solution was discarded, and 300 µL of the PBST solution was added and removed. This operation was repeated 3 times. 50 µL of a solution of Streptavidin-HRP (#ab7403, manufactured by Abcam) diluted with the PBST solution to 200 ng/mL was added, and the mixture was left standing at room temperature for 30 min. The solution was discarded, and 300 µL of the PBST solution was added and removed. This operation was repeated 3 times. 100 µL of a substrate solution (#52-00-03, manufactured by KPL) was added, and the mixture was left standing at room temperature for 15 min. Finally, 100 µL of stop solution (#50-85-06, manufactured by KPL) was added, and the absorbance at 450 nm was measured. The concentration of TSG-6 contained in each sample was calculated from 4-parameter logistic regression of the calibration curve. To calculate the amount of secretion per number of cells, a relative value was calculated by dividing the calculated amount of TSG-6 by the luminescence intensity.

As a result, the relative value of the amount of TSG-6 secretion increased more in the 3D culture in which the medium composition added with chitin nanofiber and chitosan nanofiber and the low adhesive plate were combined than in the monolayer culture using the no addition medium composition and the adhesive plate. Also, in the comparison of control and TNF-α treatment in each sample, the increase rate in the 3D culture in which the medium composition added with chitin nanofiber and chitosan nanofiber and the low adhesive plate were combined was higher than that in the monolayer culture using the no addition medium composition and the adhesive plate. The relative values of the human bone marrow-derived mesenchymal stem cells and human umbilical cord-derived mesenchymal stem cells in each sample are shown in Table 17 and Table 18.

**[Table 17]**

| human bone marrow-derived mesenchymal stem cell | | | relative value (TSG-6/Celltiter glo) | increase rate |
|---|---|---|---|---|
| adhesive plate | no addition (sample 12) | control | 1.04E-06 | 1 |
| | | TNF-α 10 ng/mL | 2.95E-06 | 2.8 |
| low adhesive plate | sample 6 | control | 4.73E-06 | 1 |
| | | TNF-α 10 ng/mL | 3.09E-05 | 6.5 |

**[Table 18]**

| human umbilical cord-derived mesenchymal stem cell | | | relative value (TSG-6/Celltiter glo) | increase rate |
|---|---|---|---|---|
| adhesive plate | no addition (sample 12) | control | 2.98E-06 | 1 |
| | | TNF-α 20 ng/mL | 7.32E-06 | 2.5 |
| low adhesive plate | sample 6 | control | 7.59E-06 | 1 |
| | | TNF-α 20 ng/mL | 3.22E-05 | 4.2 |

### (Experimental Example 11: Amount of VEGF produced by 3D culture derived from human bone marrow by using mixture of chitin nanofiber and chitosan nanofiber)

A medium composition was prepared by adding chitin nanofiber and chitosan nanofiber to mesenchymal stem cell proliferation medium 2 medium (C-28009, manufactured by Takara Bio Inc.) (final concentration: 0.05%(w/v)) (sample 6), and a no addition medium composition (sample 12) which does not containing the above-mentioned base material was prepared. Successively, the cultured human bone marrow-derived mesenchymal stem cells (C-12974, manufactured by Takara Bio Inc.) were seeded at 100000 cells/mL in the above-mentioned medium composition added with chitin and chitosan nanofiber or no addition medium composition, and the medium composition added with chitin and chitosan nanofiber was dispensed in a 24-well flat bottom ultra low attachment surface microplate (#3473, manufactured by Corning Incorporated), and the no addition medium composition was seeded in the wells of 24-well flat bottom attachment surface microplate (#3526, manufactured by Corning Incorporated), each at 1 mL per well. Each plate was cultured in a standing still state in a CO₂ incubator (37°C, 5%CO₂) continuously for 2 days. On day 2, the medium composition added with chitin and chitosan nanofiber and no addition medium composition were transferred from each well to a 15 mL tube, centrifuged at 300xg for 3 min, and the culture supernatant was removed. Successively, 1 mL of 17% FBS-containing MEMα medium was added, and the medium composition added with chitin and chitosan nanofiber was placed back in the wells of 24-well flat bottom ultra low attachment surface microplate (#3473, manufactured by Corning Incorporated). In the no addition medium composition, after removal of the mesenchymal stem cell proliferation medium 2 medium, 1 mL of the 17% FBS-containing MEMα medium was added to the 24-well flat bottom attachment surface microplate (#3526, manufactured by Corning Incorporated). Each plate was cultured in a CO₂ incubator (37°C, 5% CO₂) in a static state for 1 day. On day 1, the medium composition added with chitin and chitosan nanofiber and no addition medium composition were transferred from each well to a 15 mL tube, centrifuged at 300xg for 3 min, and the culture supernatant was recovered. To evaluate the cell number here, an ATP reagent (1 mL, CellTiter-Glo (registered trade mark) Luminescent Cell Viability Assay, manufactured by Promega) was added and suspended in each sample after recovery of the culture supernatant, and the suspension was stood for about 10 min at room temperature. The luminescence intensity (RLU value) was measured by Enspire (manufactured by Perkin Elmer) and the number of viable cells was measured by subtracting the luminescence value of the medium alone. Successively, VEGF contained in the recovered culture supernatant was quantified using a VEGF ELISA165 kit, Human (#ENZ-KIT156-0001, manufactured by Enzo Lifescience). A standard or sample solution (100 µL) was added to a well and shaken for 60 min at room temperature. Successively, the solution was discarded, 300 µL of 1x wash solution was added, and removed. The above-mentioned operation was repeated 3 times. 100 µL of VEGF detector antibody was added, and the mixture was shaken for 30 min at room temperature. The solution was discarded, 300 µL of 1x wash solution was added, and removed. The above-mentioned operation was repeated 3 times. 100 µL of VEGF conjugate (blue) was added, and the mixture was shaken for 30 min at room temperature. Successively, the solution was discarded, 300 µL of 1x wash solution was added, and removed. The above-mentioned operation was repeated 4 times. 100 µL of TMB solution was added, and the mixture was shaken for 30 min at room temperature. Finally, 100 µL of stop solution 2 was added, and the absorbance at 450 nm was measured. The concentration of VEGF contained in each sample was calculated from 4-parameter logistic regression of the calibration curve. To calculate the amount of secretion per number of cells, a relative value was calculated by dividing the calculated amount of VEGF by the luminescence intensity.

As a result, the VEGF production amount per cell increased more in the medium composition added with chitin nanofiber and chitosan nanofiber than in the culture in the no addition medium composition. The relative values of each sample are shown in Table 19.

**[Table 19]**

| | | relative value (VEGF/Celltiter glo) |
|---|---|---|
| adhesive plate | no addition (sample 12) | 0.00049 |
| low adhesive plate | sample 6 | 0.00115 |

### (Experimental Example 12: Stem cell marker expression variation of human umbilical cord-derived mesenchymal stem cell by 3D culture using mixture of chitin nanofiber and chitosan nanofiber or microcarrier)

A medium composition obtained by adding the sample 6 obtained above to a mesenchymal stem cell proliferation medium 2 medium (C-28009, manufactured by Takara Bio Inc.) to a final concentration of 0.05%(w/v) was prepared. Corning low concentration SynthemaxII microcarrier (manufactured by Corning, 3781) (360 mg) was weighed, hydrated with sterile purified water, and immersed in 10 mL of ethanol for 30 min. Thereafter, ethanol was removed, they were washed twice with 30 mL of PBS(-), and finally washed with 10 mL of mesenchymal stem cell proliferation medium 2 medium. The above-mentioned microcarriers were added to 10 mL of mesenchymal stem cell proliferation medium 2 medium to prepare the medium composition as a comparison control (sample 13). Successively, cultured human umbilical cord-derived mesenchymal stem cells (C-12971, manufactured by Takara Bio Inc.) (600000 cells) were suspended in 40 mL of the above-mentioned nanofiber-containing mesenchymal stem cell proliferation medium, and the suspension was filled in Antibody Immobilization Bag A (manufactured by Nipro, 87-362) used as a culture bag and cultured in a CO₂ incubator (37°C, 5%CO₂) in a static state for 3 days. In addition, the cultured human umbilical cord-derived mesenchymal stem cells (600000 cells) were suspended in 15 mL of a microcarrier-containing mesenchymal stem cell proliferation medium, the suspension was transferred to a Corning 125 mL disposable spinner flask, and cultured in a CO₂ incubator (37°C, 5%CO₂) in a static state for one day. One day later, the cells were further cultured for 2 days by a method using a Microstir Slow Speed Magnetic Stirrer (manufactured by WHEATON, W900701-B) and shaking at 30 rpm for 15 min, followed by standing still for 2 hr. After 3 days, the cells cultured in the Antibody Immobilization Bag A and disposable spinner flask were subjected to half-volume medium exchange, and further cultured for 4 days under the same conditions as described above. After 4 days, 1 mL was removed from 40 mL, transferred to a 1.5 mL tube, and centrifuged at 300xg for 3 min. The supernatant was removed, and RLT solution (350 µL, RNeasy mini kit (manufactured by QIAGEN, #74106)) was added to give an RNA extraction solution. Successively, to the RNA extraction solution was added 70% ethanol (350 µL), and the mixture was added to RNeasy spin column and centrifuged at 8000xg for 15 sec. Successively, 700 µL of RW1 solution was added to RNeasy spin column, and centrifuged at 8000xg for 15 sec. Successively, 500 µL of RPE solution was added, and centrifuged at 8000xg for 15 sec. Furthermore, 500 µL of RPE solution was added, and centrifuged at 8000xg for 2 min. RNase-free solution was added to RNAs remaining in the RNeasy spin column to elute them. Then, cDNAs were synthesized from the obtained RNAs by using PrimeScript RT reagent Kit (Perfect Real Time) (manufactured by Takara Bio Inc., #RR037A). Using the synthesized cDNAs, Premix EX Taq (Perfect Real Time) (manufactured by Takara Bio Inc., #RR039A), and Taq man Probe (manufactured by Applied Bio Systems), real-time PCR was performed. As the Taq man Probe (manufactured by Applied Bio Systems), OCT4 used was Hs04260367_gH, SOX2 used was Hs01053049_s1, NANOG used was Hs04399610_g1, CXCR4 used was Hs00607978 s1, and GAPDH used was Hs99999905_m1. As the instrument, real-time PCR7500 was used. In the analysis, relative values obtained by amending the values of each target gene with the values of GAPDH were calculated and compared.

As a result, when human umbilical cord-derived mesenchymal stem cells were cultured in a medium composition containing a mixture of chitin nanofiber and chitosan nanofiber in a culture bag, an increase in the relative expression levels of OCT4, SOX2, and NANOG genes showing an undifferentiated state and CXCR4 gene showing homing-related chemotacticity was observed. On the other hand, a clear increase in the expression level of these genes was not observed under the conditions of culture using Corning low concentration SynthemaxII microcarrier. The relative values of each gene expression are shown in Table 20.

**[Table 20]**

| | | SOX2 | OCT4 | NANOG | CXCR4 |
|---|---|---|---|---|---|
| day 0 | | 1.3 | 1.9 | 1.5 | 2 |
| day 7 | sample 13 | 0.1 | 0.2 | 0.4 | 0.3 |
| | sample 6 | 383.9 | 418.3 | 376.1 | 338.4 |

### (Experimental Example 13: Amount of bFGF produced by 3D culture of human adipose-derived mesenchymal stem cell using mixture of chitin nanofiber and chitosan nanofiber or microcarrier)

A medium composition obtained by adding the sample 6 obtained above to a mesenchymal stem cell proliferation medium 2 medium (C-28009, manufactured by Takara Bio Inc.) to a final concentration of 0.05%(w/v) was prepared. Corning low concentration SynthemaxII microcarrier (manufactured by Corning, 3781) (360 mg) was weighed, hydrated with sterile purified water, and immersed in 10 mL of ethanol for 30 min. Thereafter, ethanol was removed, they were washed twice with 30 mL of PBS(-), and finally washed with 10 mL of mesenchymal stem cell proliferation medium 2 medium. The above-mentioned microcarriers were added to 10 mL of mesenchymal stem cell proliferation medium 2 medium to prepare the medium composition as a comparison control (sample 13). Successively, cultured human adipose-derived mesenchymal stem cells (C-12977, manufactured by Takara Bio Inc.) (600000 cells) were suspended in 40 mL of the above-mentioned nanofiber-containing mesenchymal stem cell proliferation medium, and the suspension was filled in Antibody Immobilization Bag A (manufactured by Nipro, 87-362) used as a culture bag and cultured in a CO₂ incubator (37°C, 5%CO₂) in a static state for 3 days. In addition, the cultured human adipose-derived mesenchymal stem cells (600000 cells) were suspended in 15 mL of a microcarrier-containing mesenchymal stem cell proliferation medium, the suspension was transferred to a Corning 125 mL disposable spinner flask, and cultured in a CO₂ incubator (37°C, 5%CO₂) in a static state for one day. One day later, the cells were further cultured for 2 days by a method using a Microstir Slow Speed Magnetic Stirrer (manufactured by WHEATON, W900701-B) and shaking at 30 rpm for 15 min, followed by standing still for 2 hr. After 3 days, 1 mL was removed from 40 mL in the culture bag and spinner flask, transferred to a 1.5 mL tube and centrifuged at 300xg for 3 min. After the centrifugation, the supernatant was removed, 2 mL of PBS(-) was added for washing. After the washing, the mixture was centrifuged at 300xg for 3 min. PBS(-) was removed, they were suspended in 1 mL of 17% FBS-containing MEMα medium (manufactured by FUJIFILM Wako Pure Chemical Corporation, 135-15175), added to a 24-well flat bottom ultra low attachment surface microplate (#3473, manufactured by Corning Incorporated), and cultured for one day in a CO₂ incubator (37°C, 5%CO₂) in a static state. One day later, the culture medium was transferred to a 1.5 mL tube, centrifuged at 300xg for 3 min, and the culture supernatant was recovered in a new 1.5 mL tube. To evaluate the cell number here, an ATP reagent (1 mL, CellTiter-Glo (registered trade mark) Luminescent Cell Viability Assay, manufactured by Promega) was added and suspended in each sample after recovery of the culture supernatant, and the suspension was allowed to stand for 10 min at room temperature. The luminescence intensity (RLU value) was measured by Enspire (manufactured by Perkin Elmer), and the number of viable cells was measured by subtracting the luminescence value of the medium only. Successively, bFGF contained in the recovered culture supernatant was quantified using bFGF ELISA kit (#ELH-bFGF-1, manufactured by RayBiotech). 100 µL of a standard or sample solution was added to the well, and the mixture was shaken for 2.5 hr at room temperature. Successively, the solution was discarded, and 300 µL of 1x wash solution was added and removed. The above-mentioned operation was repeated 4 times. 100 µL of 1x Detection antibody was added, and the mixture was shaken for 1 hr at room temperature. The solution was discarded, and 300 µL of 1x wash solution was added and removed. The above-mentioned operation was repeated 4 times. 100 µL of HRP-Streptavidin solution was added, and the mixture was shaken for 45 min at room temperature. The solution was discarded, and 300 µL of 1x wash solution was added and removed. The above-mentioned operation was repeated 4 times. 100 µL of TMB One-Step Substrate reagent was added, and the mixture was shaken for 30 min at room temperature in a dark place. Lastly, 50 µL of stop solution was added, and the absorbance at 450 nm was measured. The concentration of bFGF contained in each sample was calculated from 4-parameter logistic regression of the calibration curve. To calculate the amount of secretion per number of cells, a relative value was calculated by dividing the calculated amount of bFGF by the luminescence intensity.

As a result, the relative value of the amount of bFGF secretion increased more in the 3D culture in which human adipose-derived mesenchymal stem cells were cultured in a medium composition added with chitin nanofiber and chitosan nanofiber than in the 3D culture using a medium composition added with microcarrier. The results are shown in Table 21.

**[Table 21]**

| | |
|---|---|
| | relative value (bFGF/Celltiter glo) |
| sample 13 | 0.0004 |
| sample 6 | 0.0118 |

### (Experimental Example 14: Stem cell marker expression variation of human adipose-derived mesenchymal stem cell by 3D culture using mixture of chitin nanofiber and chitosan nanofiber produced by simultaneous fibrillating or poly(1,4)-N-acetyl-β-D-glucosamine nanofiber having specific acetylation degree)

Chitin powder and chitosan powder were mixed at a weight ratio of 1:4, and the mixture was subjected to nanofiberization under conditions of 200 MPa, pass 5 times to prepared α chitin/chitosan nanofibers (hereinafter the mixture of chitin nanofiber and chitosan nanofiber produced by the present method is sometimes referred to as "chitin/chitosan nanofiber prepared by simultaneous fibrillating", etc.). Also, the poly(1,4)-N-acetyl-β-D-glucosamine nanofiber having a specific acetylation degree (about 50% in this Experimental Example) (hereinafter sometimes referred to as "nanofiber with adjusted amount of N-acetylglucosamine", etc.) was prepared by adjusting the N-acetylglucosamine amount to about 50% by heating under reflux poly(1,4)-N-acetyl-β-D-glucosamine in a 30% sodium hydroxide aqueous solution for 4 hr, and nanofiberizing same under the conditions of 200 MPa, pass 5 times. The thus-obtained chitin/chitosan nanofibers prepared by simultaneous fibrillating and nanofiber with adjusted amount of N-acetylglucosamine were each diluted with water for injection (distillation water) to 1%(w/w), and dispersed by inversion mixing, and the water suspension was subjected to an autoclave sterilization treatment at 121°C for 20 min (sample 14 and sample 15, respectively). A medium composition was prepared by adding sample 14 or sample 15 obtained above to mesenchymal stem cell proliferation medium 2 medium (C-28009, manufactured by Takara Bio Inc.) to a final concentration of 0.05%(w/v).

(1) Medium composition containing sample 14 (chitin/chitosan nanofiber prepared by simultaneous fibrillating: 0.05%(w/v)
(2) Medium composition containing sample 15 (nanofiber with adjusted amount of N-acetylglucosamine (50%): 0.05%(w/v))

Successively, the cultured human adipose-derived mesenchymal stem cells (C-12977, manufactured by Takara Bio Inc.) were proliferated by 2D culture in a petri dish, seeded in the above-mentioned medium composition added with nanofiber or no addition medium composition at 50000 cells/mL, and dispensed in the wells of a 24-well flat bottom ultra low attachment surface microplate (#3473, manufactured by Corning Incorporated) at 1.2 mL per well. Each plate was cultured in a standing still state in a CO₂ incubator (37°C, 5%CO₂) continuously for 13 days. On seeding and days 8 and 13, the cells were recovered, and RLT solution (300 µL, RNeasy mini kit (manufactured by QIAGEN, #74106)) was added to give an RNA extraction solution. To the RNA extraction solution was added 70% ethanol (300 µL), and the mixture was added to RNeasy spin column and centrifuged at 8000xg for 15 sec. Successively, 600 µL of RW1 solution was added to RNeasy spin column, and centrifuged at 8000xg for 15 sec. Successively, 500 µL of RPE solution was added, and centrifuged at 8000xg for 15 sec. Furthermore, 500 µL of RPE solution was added, and centrifuged at 8000xg for 2 min. RNase-free solution was added to RNAs remaining in the RNeasy spin column to elute them. Then, cDNAs were synthesized from the obtained RNAs by using PrimeScript RT reagent Kit (Perfect Real Time) (manufactured by Takara Bio Inc., #RR037A). Using the synthesized cDNAs, Premix EX Taq (Perfect Real Time) (manufactured by Takara Bio Inc., #RR039A), and Taq man Probe (manufactured by Applied Bio Systems), real-time PCR was performed. As the Taq man Probe (manufactured by Applied Bio Systems), OCT4 used was Hs04260367_gH, NANOG used was Hs04399610_g1, and PPIA used was Hs99999904_m1. As the instrument, real-time PCR7500 was used. In the analysis, relative values obtained by amending the values of each target gene with the values of PPIA were calculated and compared.

As a result, when human adipose-derived mesenchymal stem cells were 3D cultured in a medium composition containing chitin/chitosan nanofiber prepared by simultaneous fibrillating or nanofiber with adjusted amount of N-acetylglucosamine in a 24-well flat bottom ultra low attachment surface microplate, an increase in the relative expression levels of NANOG and OCT4 genes showing an undifferentiated state was observed as compared with the 2D culture conditions on seeding. The relative values of NANOG gene expression are shown in Table 22, and the relative values of OCT4 gene expression are shown in Table 23.

**[Table 22]**

| NANOG/PPIA | on seeding | day 8 (24 well) | day 13 (24 well) |
|---|---|---|---|
| sample 14 | 0.07 | 3.9 | 2.24 |
| sample 15 | 0.07 | 9.41 | 1.91 |

**[Table 23]**

| OCT4/PPIA | on seeding | day 8 (24 well) | day 13 (24 well) |
|---|---|---|---|
| sample 14 | 0.13 | 3.08 | 1.68 |
| sample 15 | 0.13 | 8.38 | 2.37 |

### (Experimental Example 15: Proliferation and stem cell marker expression variation of human adipose-derived mesenchymal stem cell by 3D culture using mixture of chitin nanofiber and chitosan nanofiber or mixture of chitin nanofiber and other polysaccharides)

Methylcellulose powder (1.0 g) was added to water (100 mL) and suspended by stirring, and the suspension was subjected to an autoclave sterilization treatment (121°C, 20 min.). To the obtained 1.0%(w/v) methylcellulose aqueous solution (8 mL) was added sample 1 (2 mL) and mixed by pipetting to prepare a mixture of 0.2%(w/v) chitin nanofiber and 0.8%(w/v) methylcellulose (sample 16).

Deacylated gellan gum powder (0.8 g) was added to water (100 mL), dissolved by stirring, and subjected to an autoclave sterilization treatment (121°C, 20 min) to give 0.8%(w/v) deacylated gellan gum aqueous solution. The deacylated gellan gum aqueous solution (12.5 mL) was mixed with D-PBS(+) (containing Ca, Mg) (27.5 mL) using a medium production kit (Nissan Chemical FCeM (registered trade mark)-series Preparation Kit). To the obtained 0.25%(w/v) deacylated gellan gum divalent cation crosslinked structure dispersion (8 mL) was added sample 1 (2 mL) and mixed by pipetting to prepare a mixture of 0.2%(w/v) chitin nanofiber and 0.2%(w/v) deacylated gellan gum (sample 17).

Sodium alginate powder (1.0 g) was added to water (50 mL), dissolved by stirring, and subjected to an autoclave sterilization treatment (121°C, 20 min) to give 2.0%(w/v) sodium alginate aqueous solution. The sodium alginate aqueous solution (10 mL) was mixed with D-PBS(+) (containing Ca, Mg) (10 mL) using a medium production kit (Nissan Chemical FCeM (registered trade mark)-series Preparation Kit). To the obtained 1.0%(w/v) sodium alginate divalent cation crosslinked structure dispersion (8 mL) was added sample 1 (2 mL) and mixed by pipetting to prepare a mixture of 0.2%(w/v) chitin nanofiber and 0.8%(w/v) alginic acid (sample 18).

Then, medium compositions were prepared by adding samples 6, 16, 17 and 18 obtained above to mesenchymal stem cell proliferation medium 2 medium (C-28009, manufactured by Takara Bio Inc.) to a final concentration of 0.05%(w/v). The final concentrations of the chitin nanofiber, chitosan nanofiber and other polysaccharides (methylcellulose, deacylated gellan gum, alginic acid) in the prepared medium compositions are as follows:
(1) Medium composition containing sample 6 (chitin nanofiber:0.01%(w/v), chitosan nanofiber:0.04%(w/v))
(2) Medium composition containing sample 16 (chitin nanofiber:0.01%(w/v), methylcellulose:0.04%(w/v))
(3) Medium composition containing sample 17 (chitin nanofiber:0.01%(w/v), deacylated gellan gum:0.04%(w/v))
(4) Medium composition containing sample 18 (chitin nanofiber:0.01%(w/v), alginic acid:0.04%(w/v))

Successively, the cultured human adipose-derived mesenchymal stem cells (C-12977, manufactured by Takara Bio Inc.) were seeded at 13333 cells/mL in a medium composition in which chitosan nanofiber or other polysaccharide (methylcellulose, deacylated gellan gum, alginic acid) is added to the above-mentioned chitin nanofiber, and dispensed in the wells of a 24-well flat bottom ultra low attachment surface microplate (#3473, manufactured by Corning Incorporated) at 1.2 mL per well. Each plate was cultured in a CO₂ incubator (37°C, 5%CO₂) in a static state, and culture was continued for 14 days. On seeding (day 0) and days 7, 14 after seeding, each culture medium was suspended and 300 µL was dispensed. An ATP reagent (300 µL, CellTiter-Glo^{™} Luminescent Cell Viability Assay, manufactured by Promega) was added and suspended, and the suspension was stood for about 10 min at room temperature. The luminescence intensity (RLU value) was measured by FlexStation3 (manufactured by Molecular Devices) and the luminescence value of the medium alone was subtracted to calculate the number of viable cells.

On days 7 and 14, the cells were recovered, and RLT solution (300 µL, RNeasy mini kit (manufactured by QIAGEN, #74106)) was added to give an RNA extraction solution. To the RNA extraction solution was added 70% ethanol (300 µL), and the mixture was added to RNeasy spin column and centrifuged at 8000xg for 15 sec. Successively, 600 µL of RW1 solution was added to RNeasy spin column, and centrifuged at 8000xg for 15 sec. Successively, 500 µL of RPE solution was added, and centrifuged at 8000xg for 15 sec. Furthermore, 500 µL of RPE solution was added, and centrifuged at 8000xg for 2 min. RNase-free solution was added to RNAs remaining in the RNeasy spin column to elute them. Then, cDNAs were synthesized from the obtained RNAs by using PrimeScript RT reagent Kit (Perfect Real Time) (manufactured by Takara Bio Inc., #RR037A). Using the synthesized cDNAs, Premix EX Taq (Perfect Real Time) (manufactured by Takara Bio Inc., #RR039A), and Taq man Probe (manufactured by Applied Bio Systems), real-time PCR was performed. As the Taq man Probe (manufactured by Applied Bio Systems), OCT4 used was Hs04260367_gH, NANOG used was Hs04399610_g1, and PPIA used was Hs99999904_m1. As the instrument, real-time PCR7500 was used. In the analysis, relative values obtained by amending the values of each target gene with the values of PPIA were calculated and compared.

As a result, among the polysaccharides combined with α chitin nanofiber, a medium composition containing a mixture with chitosan nanofiber showed the highest proliferative capacity of human adipose-derived mesenchymal stem cells, and high expression capacity of OCT4 gene and NANOG gene showing an undifferentiated state. A medium composition containing a mixture of chitin nanofiber and any polysaccharide from methylcellulose, deacylated gellan gum, alginic acid also proliferated human adipose-derived mesenchymal stem cells and showed an improving action on the expression of OCT4 gene and NANOG gene, though weaker than those of a mixture with chitosan nanofiber. The results of the viable cells number are shown in Table 24, the results of NANOG gene expression are shown in Table 25, and the results of OCT4 gene expression are shown in Table 26.

**[Table 24]**

| cell number | on seeding | day 7 (24 well) | day 14 (24 well) |
|---|---|---|---|
| sample 6 | 1129 | 2754 | 9943 |
| sample 16 | 1129 | 154 | 1849 |
| sample 17 | 1129 | 834 | 3764 |
| sample 18 | 1129 | 3625 | 7157 |

**[Table 25]**

| NANOG/PPIA | on seeding | day 7 (24 well) | day 14 (24 well) |
|---|---|---|---|
| sample 6 | 0.5 | 5.18 | 2.62 |
| sample 16 | 0.5 | 1.24 | 1.18 |
| sample 17 | 0.5 | 0.75 | 0.5 |
| sample 18 | 0.5 | 2.12 | 0.57 |

**[Table 26]**

| OCT4/PPIA | on seeding | day 7 (24 well) | day 14 (24 well) |
|---|---|---|---|
| sample 6 | 0.52 | 4.43 | 3.44 |
| sample 16 | 0.52 | 1.7 | 0.93 |
| sample 17 | 0.52 | 1 | 0.56 |
| sample 18 | 0.52 | 2.27 | 0.74 |

### (Experimental Example 16 (Reference Example): Proliferation, maintenance of survival, dispersibility of cell aggregates of HEK293-IFNβ cells in 3D culture using mixture of chitin nanofiber and chitosan nanofiber, mixture of chitin nanofiber and chitosan nanofiber produced by simultaneous fibrillating, or poly(1,4)-N-acetyl-β-D-glucosamine nanofiber having specific acetylation degree)

A medium composition was prepared by adding the sample 1 obtained above to EMEM medium (manufactured by Wako Pure Chemical Industries, Ltd.) containing 10%(v/v) fetal bovine serum to a final concentration of 0.02%(w/v) or 0.1%(w/v). In addition, a medium composition was prepared by adding sample 6, 14 or 15 to EMEM medium (manufactured by Wako Pure Chemical Industries, Ltd.) containing 10%(v/v) fetal bovine serum to a final concentration of 0.02%(w/v). Furthermore, a no addition medium composition (sample 12) which does not contain the above-mentioned base material was prepared. The final concentrations of each nanofiber in the prepared nanofiber-containing EMEM medium containing 10%(v/v) fetal bovine serum were as the follows:
(1) Medium composition containing sample 1 (chitin nanofiber:0.02%(w/v), chitosan nanofiber:0%(w/v))
(2) Medium composition containing sample 1 (chitin nanofiber:0.1%(w/v), chitosan nanofiber:0%(w/v))
(3) Medium composition containing sample 6 (chitin nanofiber:0.02%(w/v), chitosan nanofiber:0.08%(w/v))
(4) Medium composition containing sample 14 (chitin/chitosan nanofiber prepared simultaneous fibrillating:0.1%(w/v)
(5) Medium composition containing sample 15 (nanofiber with adjusted amount of N-acetylglucosamine (50%):0.1%(w/v))

Successively, HEK293-IPMβ cells that produce cultured human IFMβ stably were suspended in each of the above-mentioned medium compositions at about 200000 cells/mL, and seeded in 24 well flat bottom ultra low attachment surface microplate (manufactured by Corning Incorporated, #3473) at 1.2 mL/well. The cells were cultured in a CO₂ incubator (37°C, 5%CO₂) in a static state for 21 days at maximum, and the medium was changed every 1 or 2 days. On seeding (day 0) and days 7, 14, 21 after seeding, each culture medium was suspended and 300 µL was dispensed. An ATP reagent (300 µL, CellTiter-Glo^{™} Luminescent Cell Viability Assay, manufactured by Promega) was added and suspended, and the suspension was stood for about 10 min at room temperature. The luminescence intensity (RLU value) was measured by FlexStation3 (manufactured by Molecular Devices) and the luminescence value of the medium alone was subtracted to calculate the number of viable cells. On days 8, 15, 21 after seeding, photographs were taken and dispersibility of cell sphere clumps was confirmed.

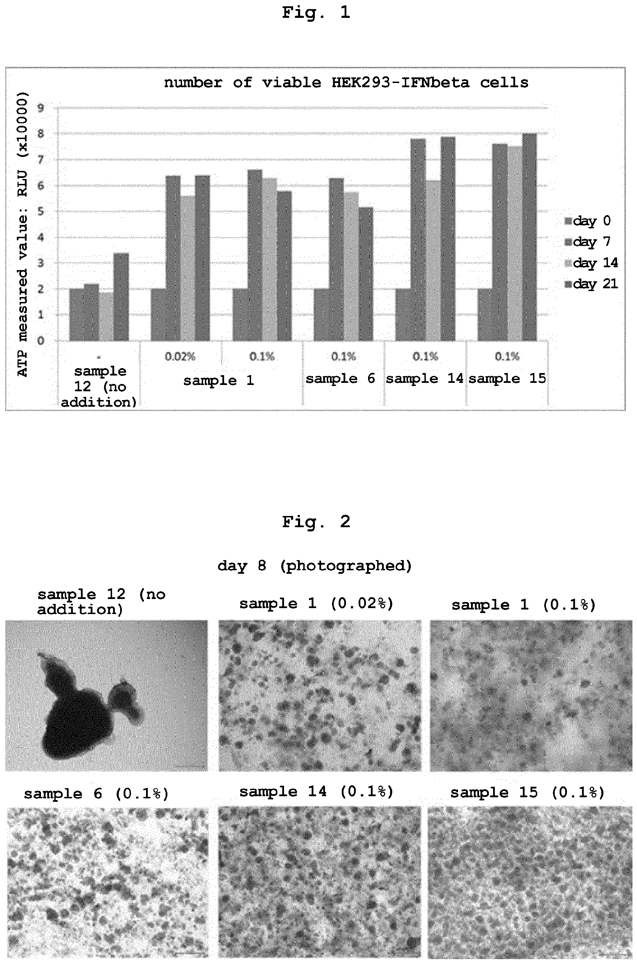

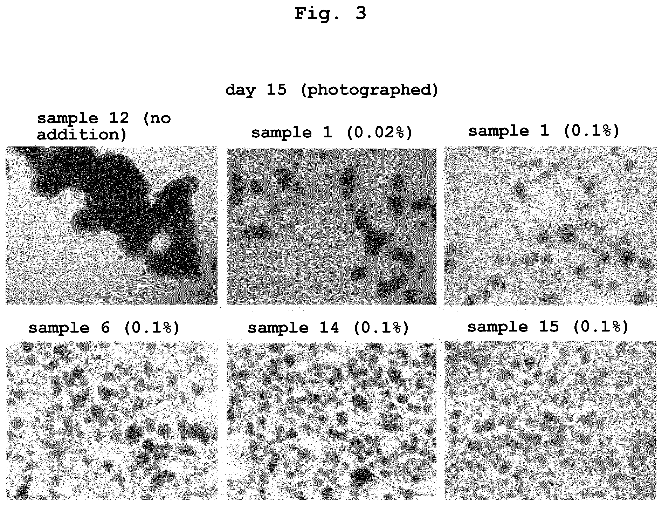

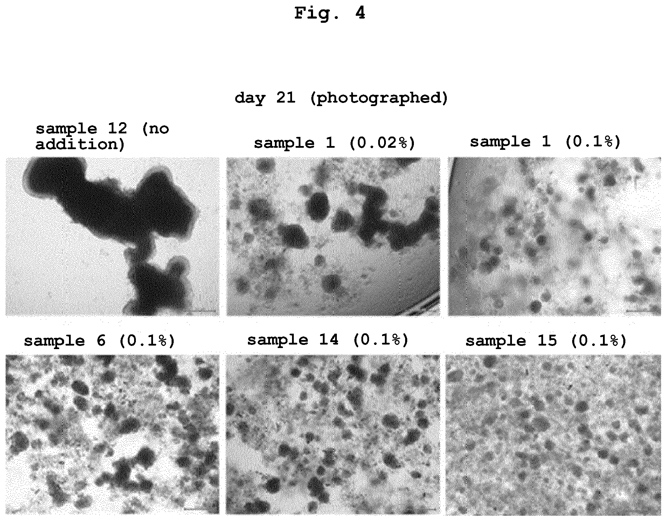

As a result, proliferation and survival maintenance effect were found under all conditions with the addition of each base material as compared with no addition conditions. When a medium composition containing chitin/chitosan nanofiber prepared by simultaneous fibrillating or nanofiber with adjusted amount of N-acetylglucosamine was used, a cell density higher than that by chitin nanofiber alone was found. By timecourse observation, a large cell aggregate was found on day 8 with no addition, cell aggregates gradually grew even with chitin nanofiber alone (sample 1), and the number of aggregates decreased. It was considered that further coagulation of cell aggregates occurred under these conditions. On the other hand, when a medium composition containing chitin nanofiber and chitosan nanofiber or a medium composition containing chitin/chitosan nanofiber prepared by simultaneous fibrillating was used, coagulation of cell aggregates was suppressed. Particularly, when a medium composition containing nanofiber with adjusted amount of N-acetylglucosamine was used, small cell aggregates tended to be dispersed even on day 21. The results of viable cells number are shown in Fig. 1, and the results of photograph observation are shown in Fig. 2 (day 8), Fig. 3 (day 15), and Fig. 4 (day 21).

### [Industrial Applicability]

According to the present invention, high-quality adherent cells (specifically, mesenchymal stem cells) can be produced efficiently. The cells obtained by the present invention are very high-quality stem cells that maintain properties such as undifferentiated state, chemotacticity and the like in a preferable state. Therefore, the stem cells obtained by the present invention (mesenchymal stem cells) can be preferably used to supplement organs and tissues lost due to diseases and the like. In addition, the cell secretion products obtained by the present invention can also be used in the treatment of various diseases. Therefore, the present invention is considered to be extremely useful in, for example, the field of regenerative medicine.

This application is based on a patent application No. 2018-164042 filed in Japan (filing date: August 31, 2018) and a patent application No. 2019-134058 filed in Japan (filing date: July 19, 2019).

## Claims

1. A medium composition for suspension culture of a mesenchymal stem cell, wherein the medium composition is a medium composition comprising
(1) a chitin nanofiber; and
(2) a chitosan nanofiber
wherein the ratio of the chitin nanofiber and the chitosan nanofiber is chitin nanofiber:chitosan nanofiber=1:3 - 6;
wherein the ratio of N-acetylglucosamine in the sugar units constituting chitin is not less than 90%; and
wherein the ratio of N-acetylglucosamine in the sugar units constituting chitosan is not more than 30%.

2. The medium composition according to claim 1, wherein the mesenchymal stem cell is a cell that self-aggregates under suspension culture.

3. The medium composition according to any one of claims 1 or 2, wherein the suspension culture is for proliferating the mesenchymal stem cell, and maintaining pluripotency and chemotacticity of the mesenchymal stem cell.

4. A method for culturing a mesenchymal stem cell, comprising a step of suspension culturing the mesenchymal stem cell in a medium composition as defined in any of claims 1 to 3.

5. The method according to claim 4, wherein the method further comprises the following steps:
(1) a step of adding the medium composition according to any one of claims 1 to 3 without performing a treatment for detaching the suspension cultured cells from the chitin nanofiber, and chitosan nanofiber, and
(2) a step of subjecting the mixture obtained in step (1) to suspension culture.

6. A method for producing a cell secretion product, comprising a step of suspension culturing the mesenchymal stem cell in a medium composition as defined in any one of claim 1 or 2.

7. The method according to claim 6 wherein a concentration of the serum in the medium composition is not more than 2%.

8. The method according to claim 6, wherein the cell secretion product is at least one selected from the group consisting of a low-molecular-weight compound, a protein, a nucleic acid, and a cell secretion vesicle.

## Patentansprüche

1. Mediumzusammensetzung für eine Suspensionskultur einer mesenchymalen Stammzelle, wobei die Mediumzusammensetzung eine Mediumzusammensetzung umfassend
(1) eine Chitin-Nanofaser; und
(2) eine Chitosan-Nanofaser ist, wobei das Verhältnis der Chitin-Nanofaser und der Chitosan-Nanofaser Chitin-Nanofaser:Chitosan-Nanofaser=1:3 - 6 ist; wobei das Verhältnis von N-Acetylglucosamin in den Zuckereinheiten, die Chitin konstituieren, nicht weniger als 90% ist; und wobei das Verhältnis von N-Acetylglucosamin in den Zuckereinheiten, die Chitosan konstituieren, nicht mehr als 30% ist.

2. Mediumzusammensetzung nach Anspruch 1, wobei die mesenchymale Stammzelle eine Zelle ist, die in der Suspensionskultur selbstaggregiert.

3. Mediumzusammensetzung nach einem der Ansprüche 1 oder 2, wobei die Suspensionskultur zum Proliferieren der mesenchymalen Stammzelle und zum Erhalten der Pluripotenz und Chemotaktizität der mesenchymalen Stammzelle dient.

4. Verfahren zum Kultivieren einer mesenchymalen Stammzelle, umfassend einen Schritt des Suspensionskultivierens der mesenchymalen Stammzelle in einer Mediumzusammensetzung, wie in einem der Ansprüche 1 bis 3 definiert.

5. Verfahren nach Anspruch 4, wobei das Verfahren ferner die folgenden Schritte umfasst:
(1) einen Schritt des Zugebens der Mediumzusammensetzung nach einem der Ansprüche 1 bis 3 ohne Durchführen einer Behandlung zum Ablösen der kultivierten Suspensionszellen von der Chitin-Nanofaser und der Chitosan-Nanofaser, und
(2) einen Schritt des Unterwerfens der in Schritt (1) erhaltenen Mischung einer Suspensionskultur.

6. Verfahren zur Herstellung eines Zellsekretionsprodukts, umfassend einen Schritt des Suspensionskultivierens der mesenchymalen Stammzelle in einer Mediumzusammensetzung, wie in einem der Ansprüche 1 oder 2 definiert.

7. Verfahren nach Anspruch 6, wobei eine Konzentration des Serums in der Mediumzusammensetzung nicht mehr als 2% beträgt.

8. Verfahren nach Anspruch 6, wobei das Zellsekretionsprodukt mindestens eines ist, das aus der Gruppe bestehend aus einer niedermolekularen Verbindung, einem Protein, einer Nukleinsäure und einem Zellsekretionsvesikel ausgewählt ist.

## Revendications

1. Composition de milieu pour culture en suspension d'une cellule souche mésenchymateuse, laquelle composition de milieu est une composition de milieu comprenant
(1) des nanofibres de chitine ; et
(2) des nanofibres de chitosane
dans laquelle le rapport des nanofibres de chitine aux nanofibres de chitosane est nanofibres de chitine / nanofibres de chitosane = 1 / 3 à 6;
dans laquelle la proportion de N-acétylglucosamine dans les motifs sucre constituant la chitine n'est pas inférieure à 90 % ; et
dans laquelle la proportion de N-acétylglucosamine dans les motifs sucre constituant le chitosane n'est pas supérieure à 30 %.

2. Composition de milieu selon la revendication 1, dans laquelle la cellule souche mésenchymateuse est une cellule qui s'auto-agrège en culture en suspension.

3. Composition de milieu selon l'une quelconque des revendications 1 et 2, dans laquelle la culture en suspension est destinée à une prolifération de la cellule souche mésenchymateuse, et au maintien de la pluripotence et du chimiotactisme de la cellule souche mésenchymateuse.

4. Procédé pour cultiver une cellule souche mésenchymateuse, comprenant une étape de culture en suspension de la cellule souche mésenchymateuse dans une composition de milieu telle que définie dans l'une quelconque des revendications 1 à 3.

5. Procédé selon la revendication 4, lequel procédé comprend en outre les étapes suivantes :
(1) une étape d'addition de la composition de milieu selon l'une quelconque des revendications 1 à 3 sans mise en oeuvre d'un traitement pour détacher les cellules cultivées en suspension des nanofibres de chitine, et des nanofibres de chitosane, et
(2) une étape de soumission du mélange obtenu dans l'étape (1) à une culture en suspension.

6. Procédé pour produire un produit de sécrétion cellulaire, comprenant une étape de culture en suspension de la cellule souche mésenchymateuse dans une composition de milieu telle que définie dans l'une quelconque des revendications 1 et 2.

7. Procédé selon la revendication 6, dans lequel la concentration du sérum dans la composition de milieu n'est pas supérieure à 2 %.

8. Procédé selon la revendication 6, dans lequel le produit de sécrétion cellulaire est au moins l'un choisi dans le groupe constitué par un composé de faible poids moléculaire, une protéine, un acide nucléique, et une vésicule de sécrétion cellulaire.
